# EUROPÄISCHE PATENTSCHRIFT

(11) **EP 1 108 037 B1**
(45) Veröffentlichungstag und Bekanntmachung des Hinweises auf die Patenterteilung: **22.10.2008**
(21) Anmeldenummer: 99968682.7
(22) Anmeldetag: 27.08.1999
(51) Int. Cl.: C12N 15/52, C12N 9/10, C12N 15/31, C12N 5/10, C12N 15/82, C12P 19/18

(54) **NUCLEINSÄUREMOLEKÜLE CODIEREND ENZYME, DIE FRUCTOSYLTRANSFERASEAKTIVITÄT BESITZEN UND DEREN VERWENDUNG**
NUCLEIC ACID MOLECULES WHICH CODE FOR ENZYMES WITH FRUCTOSYLTRANSFERASE ACTIVITY AND USE THEREOF
ENZYMES CODANT DES MOLECULES D'ACIDE NUCLEIQUE ET AYANT UNE ACTIVITE DE TRANSFERASE DU FRUCTOSYLE, ET UTILISATION DE CES ENZYMES

(30) Priorität: 02.09.1998 DE 19840028
(43) Veröffentlichungstag der Anmeldung: 20.06.2001
(73) Patentinhaber: MAX-PLANCK-GESELLSCHAFT ZUR FÖRDERUNG DER WISSENSCHAFTEN E.V., 14195 Berlin (DE)
(72) Erfinder: HEYER, Arnd, Göran, 70567 Stuttgart (DE); REHM, Jochen, 84051 Altheim (DE); WENDENBURG, Regina, 14059 Berlin (DE)
(74) Vertreter: Vossius & Partner
(86) Internationale Anmeldenummer: PCT/EP1999/006319
(87) Internationale Veröffentlichungsnummer: WO 2000/014246

(56) Entgegenhaltungen:
- EP-A- 0 307 158
- WO-A-94/14970
- DE-A- 19 617 687
- SOMIARI ET AL.: "Cloning and sequencing of an Aspergillus niger gene coding for beta-fructofuranosidase" EMBL SEQUENCE DATABASE, 14. November 1997 (1997-11-14), XP002127822 HEIDELBERG DE -& SOMIARI ET AL.: EMBL SEQUENCE DATABASE, 1. Januar 1998 (1998-01-01), XP002127823 HEIDELBERG DE & SOMIARI ET AL.: BIOTECHNOL LETT, Bd. 19, Nr. 12, 1997, Seiten 1243-1247,
- PATENT ABSTRACTS OF JAPAN vol. 017, no. 135 (C-1037), 19. März 1993 (1993-03-19) & JP 04 311378 A (AJINOMOTO CO INC), 4. November 1992 (1992-11-04)

## Beschreibung

Die vorliegende Erfindung betrifft Nucleinsäuremoleküle, die Polypeptide mit der enzymatischen Aktivität einer Fructosyltransferase codieren. Weiterhin betrifft diese Erfindung Vektoren und Wirte, die derartige Nucleinsäuremoleküle enthalten. Die vorliegende Erfindung betrifft ebenfalls Verfahren zur Herstellung von Fructosyltransferase und die Produktion von Polyfructosen des Inulintyps in verschiedenen Wirtsorganismen oder *in vitro,* sowie die von den beschriebenen Nucleinsäuremolekülen codierte Fructosyltransferase, mit deren Hilfe Polyfructosen des Inulintyps erzeugt werden kann.

Wasserlösliche, lineare Polymere besitzen vielfältige Anwendungen, beispielsweise zur Viskositätserhöhung von wäßrigen Systemen, als Detergenzien, als suspendierendes Agens oder zur Sedimentationsbeschleunigung und Komplexierung, aber auch zur Wasserbindung. Polymere auf der Basis von Sacchariden, beispielsweise Fructosylpolysacchariden, sind besonders interessante Rohstoffe, da sie biologisch abbaubar sind.
Neben einem Einsatz als nachwachsende Rohstoffe für industrielle Fertigung und Verarbeitung sind Fructosylpolymere aber auch als Lebensmittelzusatzstoffe, beispielsweise als Süßstoffe interessant. Für die verschiedenen Anwendungen werden Polymere unterschiedlicher Kettenlängen benötigt. Während für den Lebensmittelbereich vorzugsweise kurz- und mittelkettige Polymere erwünscht sind, verlangen technische Anwendungen, beispielsweise die Herstellung von Tensiden, Polymere mit hohem Polymerisationsgrad (DP; "Degree of Polymerisation").
Bisher sind verschiedene Verfahren zur Produktion von Fructanpolysacchariden in Pflanzen durch Expression von Fructosyltransferasen bakteriellen Ursprungs oder zur Produktion von Polyfructosen mittlerer Kettenlänge durch Expression von Fructosyltransferasen pflanzlichen Ursprungs beschrieben worden. So wird in PCT/US89/02729 die Möglichkeit der Erzeugung von Kohlenhydratpolymeren, insbesondere Dextran oder Polyfructose, in transgenen Pflanzen, und zwar speziell den Früchten transgener Pflanzen, beschrieben. Zur Erzeugung solcherart veränderter Pflanzen wird die Verwendung von Levansucrasen aus Mikroorganismen, insbesondere aus *Aerobacter levanicum, Streptococcus salivarius* und *Bacillus subtilis,* oder von Dextransucrasen aus *Leuconostoc mesenteroides* vorgeschlagen. Weder die Bildung der aktiven Enzyme, noch die von Levan oder Dextran sowie die Herstellung transgener Pflanzen wird beschrieben.
Die PCT/EP93/02110 offenbart ein Verfahren zur Herstellung transgener Pflanzen, die das lsc Gen der Levansucrase aus dem gram-negativen Bakterium *Erwinia amylovora* exprimieren. Die Pflanzen produzieren ein hochmolekulares, stark verzweigtes Levan.
In PCT/NL93/00279 wird die Transformation von Pflanzen mit chimären Genen beschrieben, die das sacB Gen aus *Bacillus subtilis* enthalten. Derartige Pflanzen produzieren ein verzweigtes Levan.
Die in der PCT/US89/02729, PCT/EP93/02110 und der PCT/NL93/00279 verwendeten bakteriellen Fructosyltransferasen synthetisieren Levan, ein β-2,6-verknüpftes Fructosylpolymer, das zahlreiche β-2,1-Verzweigungen aufweist. Levan birgt aber wegen der zahlreichen Verzweigungen entscheidende Nachteile für die technische Verarbeitung und ist daher als technischer Rohstoff weitaus weniger gefragt als Inulin, bei dem β-2,1-Verknüpfungen vorliegen. Derzeit ist nur ein bakterielles Gen bekannt, dessen Genprodukt an der Synthese von Inulin beteiligt ist. Hierbei handelt es sich um das ftf Gen aus *Streptococcus mutans.* Die PCT/NL93/00279 beschreibt die Transformation von Pflanzen mit diesem Gen, die zwar hochmolekulares Inulin synthetisieren, jedoch in so geringer Ausbeute, daß eine wirtschaftliche Nutzung nicht in Betracht kommt. Auch die PCT/EP97/02195 beschreibt ein Verfahren zur Herstellung von transgenen, Inulin erzeugenden Pflanzen mit Hilfe des ftf Gens aus *Streptococcus mutans.* Ebenso wie bei den in der PCT/NL93/00279 beschriebenen Pflanzen ist jedoch die Ausbeute an hochmolekularem Inulin gering. Eine Expression des Gens in Pflanzen ist daher zwar möglich, wenn das Gen zuvor gentechnisch bearbeitet wurde. Doch ist die Ausbeute an Inulin, das aus transgenen Pflanzen gewonnen werden kann, so gering, daß eine wirtschaftliche Nutzung der transgenen Pflanzen nicht in Betracht, kommt.
Ferner offenbart die PCT/NL96/00012 DNA-Sequenzen, die Kohlenhydratpolymer synthetisierende Enzyme codieren, sowie die Herstellung von transgenen Pflanzen mit Hilfe dieser DNA-Sequenzen. Die offenbarten Sequenzen stammen aus *Helianthus tuberosus.* Entsprechend PCT/NL96/00012 können die offenbarten Sequenzen genutzt werden, um das Fructanprofil von Petunie und Kartoffel, aber auch von *Helianthus tuberosus* selbst zu verändern. Bei Expression der offenbarten Sequenzen, die eine saccharoseabhängige Saccharose-Fructosyltransferase (SST) bzw. eine Fructan-Fructosyltransferase codieren, in transgenen Pflanzen ist die Produktion von Inulin möglich. Der durchschnittliche Polymerisationsgrad des Inulins liegt jedoch bei DP=6 bis DP=10. Bei einem solchen Polymerisationsgrad kann jedoch nicht von langkettigem Inulin gesprochen werden. Die Produktion von hochmolekularem Inulin ist mit dem in PCT/NL96/00012 beschriebenen Verfahren nicht möglich.
Vor kurzem wurde von Rehm et al. (J. Bacteriology 180 (1998), 1305-1310) die Erzeugung von Oligosacchariden in Hefe durch die Einführung einer SST aus *Aspergillus foetidus* beschrieben. Allerdings betrug der Polymerisationsgrad des erhaltenen Produkts lediglich DP=3.
Die DE 197 08 774.4 beschreibt die Herstellung von 1-Kestose und Nystose mit Hilfe von Enzymen, die Fructosylpolymeraseaktivität besitzen. Die Tri- und Tetrasaccharide können in transgenen Pflanzen hergestellt werden. Die Ausbeute ist hoch und entspricht in Kartoffel dem zellulären Gehalt an Saccharose. Eine Herstellung von längerkettigem Inulin wird jedoch nicht beschrieben.
Auch die Synthese von Polyfructosen durch Pilze wird vielfältig diskutiert. Barthomeuf und Pourrat (Biotechnology Letters 17 (1995), 911-916), beschreiben z.B. eine Enzympräparation von *Penicillium rugulosum,* die eine Fructosyltransferaseaktivität aufweist. Das Präparat hat aber verschiedene enzymatische Eigenschaften, stellt also keine reine Fructosyltransferase dar. DNA-Sequenzen des Fructosyltransferasegens sind nicht bekannt. Caims et al. (New Phytologist 129 (1995), 299-308), beschreiben eine transiente Synthese von Tri-, Tetra- und Pentasacchariden aus Saccharose im Kulturmedium von *Monographella nivalis.* Die zugrundeliegende Enzymaktivität scheint aber hauptsächlich hydrolytisch zu sein, da mit zunehmender Substratverarmung auch die Polyfructosen vom Enzym wieder abgebaut werden. Da keine DNA-Sequenz bekannt ist, kann auch nicht anhand der Homologie mit Fructofuranosidasen (Invertasen) beurteilt werden, ob eine Fructosyltransferase im eigentlichen Sinn oder eine Invertase vorliegt.
Für den Pilz *Aspergillus sydowi* IAM 2544 wurde gezeigt, daß er Polyfructosen des Inulintyps bilden kann. Harada et al. (in: Nishinari und Doi (Eds.), Food Hydrocolloids: Structures, Properties and Functions, Plenum, New York (1994), 77-82) beschreiben z.B. die Synthese von Inulin mit Konidien von *Aspergillus sydowi.* Es wurden 125 g Konidien in 25 l 20% Saccharoselösung inkubiert. Die Aufreinigung des gebildeten Produkts erfolgte durch HPLC. Ein derartiges Verfahren eignet sich aber nicht zur großtechnischen Produktion von Inulin. Weiterhin beschreiben Maramatsu et al. (Agric. Biol. Chem. 52 (1988), 1303-1304) die Produktion von Fructooligosacchariden mit Mycel des gleichen Pilzstammes (A. *sydowi* IAM 2544). Der Polymerisationsgrad wird mit 3 bis 13 angegeben. Die beteiligten Enzyme wurden nicht bzw. nur teilweise gereinigt. Aminosäuresequenzen oder DNA-Sequenzen der entsprechenden Gene sind nicht bekannt. Vorschriften zur Reinigung der Proteine sind nicht bzw. nur teilweise dargelegt.

Der vorliegenden Erfindung liegt somit die Aufgabe zugrunde, Nucleinsäuremoleküle und Verfahren zur Verfügung zu stellen, mit deren Hilfe es möglich ist, gentechnisch veränderte Organismen herzustellen, die Polyfructosen des Inulintyps bilden können.
Diese Aufgabe wird durch die Bereitstellung der in den Patentansprüchen gekennzeichneten Ausführungsformen gelöst.

Somit betrifft die vorliegende Erfindung Nucleinsäuremoleküle, codierend eine Fructosyltransferase, ausgewählt aus der Gruppe bestehend aus
(a) Nucleinsäuremolekülen, die ein Protein codieren, das die unter SEQ ID No. 2 angegebene Aminosäuresequenz umfaßt;
(b) Nucleinsäuremolekülen, die die Nucleotidsequenz der unter SEQ ID No. 1 angegebenen codierenden Region umfassen oder eine korrespondierende Ribonucleotidsequenz;
(c) Nucleinsäuremolekülen, die mit einem komplementären Strang der unter (a) oder (b) genannten Nucleinsäuremoleküle hybridisieren und eine Identität von mindestens 60% zu dem unter (a) oder (b) genannten Nucleinsäuremolekül aufweisen; und
(d) Nucleinsäuremolekülen, deren Nucleotidsequenz aufgrund der Degeneration des genetischen Codes von der Sequenz der unter (c) genannten Nucleinsäuremoleküle abweicht;
sowie die zu diesen komplementären Nucleinsäuremoleküle.

Die unter SEQ ID No. 1 dargestellte Sequenz codiert eine Saccharose-abhängige Fructan-Fructosyltransferase aus *Aspergillus sydowi,* die in pflanzlichen Zellen zur Synthese eines langkettigen Polyfructans vom Inulintyp führt. Es wurde überraschenderweise gefunden, daß es mit Hilfe dieser Sequenzen möglich ist, langkettige Polyfructane vom Inulintyp in hohen Ausbeuten in Wirtsorganismen, insbesondere in transgenen-Pflanzen, Bakterien oder Pilzen herzustellen.
Im Rahmen der vorliegenden Erfindung wurden Vorschriften zur Reinigung des Enzyms aus *Aspergillus sydowi* erarbeitet. Das Enzym wurde bis zur Homogenität gereinigt, um Aminosäuresequenzen ermitteln zu können. Anhand der gewonnen Sequenzinformationen wurden *primer* für eine Polymerase-Kettenreaktion ermittelt. Mit Hilfe der *primer* wurden Genfragmente amplifiziert, die zur Durchmusterung von cDNA-Bibliotheken eingesetzt wurden. Mehrere cDNA-Moleküle mit Sequenzhomologie zu den PCR-Amplifikaten wurden präpariert und verglichen. Die Mehrzahl der gewonnenen cDNA-Moleküle wies jeweils gleich große Insertionen auf. Die Vollständigkeit der cDNA-Moleküle zeigte sich bei der funktionalen Expression der DNA-Sequenzen in *Saccharomyces* bzw. in Kartoffel.
Die Reinigung der Enzyme, das Design von *primern* für die PCR, die Identifikation von cDNA-Molekülen und die heterologe Expression sind in den Beispielen beschrieben. Die isolierte DNA-Sequenz und die von ihr ableitbare Proteinsequenz sind als SEQ. ID No. 1 bzw. 2 wiedergegeben. Die erfindungsgemäße DNA-Sequenz ist die erste, die eine saccharoseabhängige Fructan-Fructosyltransferase aus Pilzen codiert. Die DNA-Sequenz und die durch sie codierte Proteinsequenz unterscheiden sich sehr stark von DNA-Sequenzen, die bereits bekannte Fructosyltransferasen codieren. So besteht beispielsweise lediglich eine Identität von 22,6% bzw. 39% zu der Fructosyltransferase von *Aspergillus naeslundii lev j* auf der Protein-bzw. DNA-Ebene. Dagegen besteht zwar eine 64 bzw. 60,6%ige Identität auf der Protein- bzw. DNA-Ebene zu einem Invertase-Gen aus *Aspergillus niger.* Das von diesem Gen codierte Protein hat jedoch eine gänzlich andere Enzymaktivität. Dies zeigt, daß es sich bei den erfindungsgemäßen Nucleinsäuremolekülen und den durch sie codierten Fructosyltransferasen um bisher nicht beschriebene Moleküle handelt.

Im Zusammenhang mit der vorliegenden Erfindung wird daher unter einer Fructosyltransferase ein Protein verstanden, das in der Lage ist, die Knüpfung von β-2,1-glycosidischen und/oder β-2,6-glycosidischen Bindungen zwischen Fructoseeinheiten zu katalysieren. Dabei stammt der zu übertragende Fructosylrest aus Saccharose.
Die durch eine erfindungsgemäße saccharoseabhängige Fructan-Fructosyltransferase katalysierte Reaktion kann folgendermaßen dargestellt werden:

n(G-F) → G - Fₙ + (n-1)G

Dabei ist G = Glucose, F = Fructose und G - F = Saccharose. D.h. das Enzym überträgt Fructosereste von Saccharose auf ein Polyfructan, das ausgehend von einem Saccharosemolekül durch β-2,1-glycosidische Verknüpfungen gebildet wird, wobei gegebenenfalls auch β-2,6-glycosidische Verknüpfungen auftreten können. Ein durch ein erfindungsgemäßes Nucleinsäuremolekül codiertes Polypeptid mit der Aktivität einer Fructosyltransferase führt zur Synthese von Polyfructose und insbesondere in Pflanzenzellen zur Synthese von Polyfructose des Inulintyps (im folgenden auch Inulin).

Im Zusammenhang mit der vorliegenden Erfindung wird unter Polyfructose ein Polyfructan mit einem Polymerisationsgrad von DP ≥ 4, vorzugsweise von DP ≥ 6, und insbesondere von DP ≥ 8 verstanden. Unter "Polyfructose des Inulintyps" oder "Inulin" wird ein langkettiges Fructanpolymer verstanden, dessen Moleküle überwiegend β-2,1-glycosidisch verknüpft sind, und gegebenenfalls auch β-2,6-Verzweigungen aufweisen. "Langkettig" bedeutet dabei, daß ein Polymerisationsgrad (DP) von mehr als 20, vorzugsweise von mehr als 50, weiterhin bevorzugt von mehr als 100 und besonders bevorzugt von mehr als 200 vorliegt. Das Fructosylpolymer kann endständig einen Glucoserest tragen, der über die C-1 OH-Gruppe der Glucose und die C-2 OH-Gruppe eines Fructosylrests verknüpft ist. In diesem Fall ist also ein Molekül Saccharose im Fructosylpolymer enthalten.
Bei Verwendung des Enzyms zur Synthese von Polyfructan *in vitro* wird ein oligomeres Produkt (DP=4 bis 10) erhalten.
Das von den erfindungsgemäßen Nucleinsäuremolekülen codierte Enzym läßt sich von bekannten Fructosyltransferasen insbesondere aufgrund der katalysierten Reaktion unterscheiden. So katalysieren die bekannten pflanzlichen SSTs die Reaktion:

G-F+G-F→G-F-F+G.

Die pflanzlichen fructanabhängigen Fructan-Fructosyltransferasen katalysieren dagegen die Reaktion:

G - Fₙ + G - Fₘ → G - Fₙ₊₁ + G-Fₘ₋₁,

wobei diese Reaktion vollständig reversibel ist.
Bakterielle Fructosyltransferasen, z.B. die durch das sacB-Gen codierte aus *Bacillus subtilis,* katalysieren ebenfalls eine Reaktion nach dem Schema

nG - F → G - Fₙ + (n-1) G.

Allerdings synthetisieren diese Enzyme Levan, d.h. ein β-2,6-glycosidisch verknüpftes Polyfructan, das β-2,1-Verzweigungen aufweist.
Das durch das *ftf*-Gen aus *Streptococcus mutans* codierte Protein (eine FTF) führt zwar auch zur Synthese von Inulin mit einem Molekulargewicht von mehreren Millionen Dalton. Das *ftf*-Gen bzw. das codierte Protein weist allerdings keine nennenswerte Homologie zu der unter SEQ ID No. 1 dargestellten Nucleinsäuresequenz (nur 37,3%) bzw. zu der unter SEQ ID No. 2 dargestellten Aminosäuresequenz auf (nur 22,6%).

Der Begriff "Hybridisierung" bedeutet im Rahmen dieser Erfindung eine Hybridisierung unter konventionellen Hybridisierungsbedingungen, vorzugsweise unter stringenten Bedingungen, wie sie beispielsweise in Sambrook et al., Molecular Cloning, A Laboratory Manual, 2. Aufl. (1989) Cold Spring Harbor Laboratory Press, Cold Spring Harbor, NY, beschrieben sind. Ein Beispiel für stringente Hybridisierungsbedingungen ist die Hybridisierung in 50% Formamid, 5 x SSC, 5 x Denhardt's-Lösung, 40 mM Natriumphosphat pH 6,8; 0,5% (Gew./Vol.) BSA, 1 % (Gew./Vol.) SDS, 0,1 mg/ml Heringsperma-DNA bei einer Hybridisierungstemperatur von 42°C und anschließendes Waschen der Filter in 0,5 x SSC/0,5% SDS bei 60°C.
Ein Beispiel für konventionelle nicht stringente Hybridisierungsbedingungen ist eine Hybridisierung unter den angegebenen Bedingungen mit der Ausnahme, daß 30% Formamid anstatt 50% verwendet werden und die Filter anschließend in 2 x SSC/0,5% SDS bei 56°C gewaschen werden. Nucleinsäuremoleküle, die mit den erfindungsgemäßen Molekülen hybridisieren, können z.B. aus genomischen oder aus cDNA-Bibliotheken isoliert werden, die vorzugsweise aus Pilzen hergestellt wurden. Die Identifizierung und Isolierung derartiger Nucleinsäuremoleküle kann dabei unter Verwendung der erfindungsgemäßen Moleküle oder Teile dieser Moleküle bzw. der reversen Komplemente dieser Moleküle erfolgen, z.B. mittels Hybridisierung nach Standardverfahren (siehe z.B. Sambrook et al., 1989, Molecular Cloning, A Laboratory Manual, 2. Aufl. Cold Spring Harbor Laboratory Press, Cold Spring Harbor, NY). Als Hybridisierungssonde können z.B. Nucleinsäuremoleküle verwendet werden, die exakt die oder im wesentlichen die unter SEQ ID No. 1 angegebene Nucleotidsequenz oder Teile dieser Sequenz aufweisen. Bei den als Hybridisierungssonde verwendeten Fragmenten kann es sich auch um synthetische Fragmente handeln, die mit Hilfe üblicher Synthesetechniken hergestellt wurden und deren Sequenz im wesentlichen mit der eines erfindungsgemäßen Nucleinsäuremoleküls übereinstimmt.

Die mit den erfindungsgemäßen Nucleinsäuremolekülen hybridisierenden Moleküle umfassen auch Fragmente, Derivate und allelische Varianten der oben beschriebenen Nucleinsäuremoleküle, die ein erfindungsgemäßes Protein codieren. Unter "Fragmenten" werden dabei Teile der Nucleinsäuremoleküle verstanden, die lang genug sind, um ein Protein mit Fructosyltransferaseaktivität zu codieren. Der Ausdruck "Derivat" bedeutet in diesem Zusammenhang, daß die Sequenzen dieser Moleküle sich von den Sequenzen der oben beschriebenen Nucleinsäuremoleküle an einer oder mehreren Positionen unterscheiden aber einen hohen Grad an Homologie zu diesen Sequenzen aufweisen. Homologie bedeutet dabei eine Sequenzidentität von mindestens 60%, vorzugsweise über 80% und besonders bevorzugt über 90%. Dabei weisen die durch diese Nucleinsäuremoleküle codierten Proteine vorzugsweise eine Sequenzidentität zu der in SEQ ID No. 2 angegebenen Aminosäuresequenz von mindestens 60%, vorzugsweise mindestens 70%, insbesondere mindestens 80%, besonders bevorzugt mindestens 90% und ganz bevorzugt von mindestens 95% auf. Die Abweichungen zu den oben beschriebenen Nucleinsäuremolekülen können dabei beispielsweise durch Deletion, Substitution, Insertion und/oder Rekombination entstanden sein. Erfindungsgemäße Nucleinsäuremoleküle können auch anderweitige Derivate der Sequenzen pilzlichen Ursprungs sein. Eine Derivatisierung der Sequenzen kann notwendig sein, um eine Expression in bestimmten Wirtszellen möglich zu machen.
Bei den Nucleinsäuremolekülen, die homolog zu den oben beschriebenen Molekülen sind und Derivate dieser Moleküle darstellen, handelt es sich in der Regel um Variationen dieser Moleküle, die Modifikationen darstellen, die dieselbe biologische Funktion ausüben. Es kann sich dabei sowohl um natürlicherweise auftretende Variationen handeln, beispielsweise um Sequenzen aus anderen Stämmen oder Organismen, oder um Mutationen, wobei diese Mutationen auf natürliche Weise aufgetreten sein können oder durch gezielte Mutagenese eingeführt wurden. Ferner kann es sich bei den Variationen um synthetisch hergestellte Sequenzen handeln. Bei den allelischen Varianten kann es sich sowohl um natürlich auftretende Varianten handeln, als auch um synthetisch hergestellte oder durch rekombinante DNA-Techniken erzeugte Varianten.

Die von den verschiedenen Varianten der erfindungsgemäßen Nucleinsäuremoleküle codierten Proteine weisen vorzugsweise bestimmte gemeinsame Charakteristika auf, wie Enzymaktivität, Molekulargewicht, immunologische Reaktivität oder Konformation, oder physikalische Eigenschaften, wie das Laufverhalten in Gelelektrophoresen, chromatographisches Verhalten, Sedimentationskoeffizienten, Löslichkeit, spektroskopische Eigenschaften, Stabilität, pH-Optimum oder Temperatur-Optimum.

In einer bevorzugten Ausführungsform codieren die erfindungsgemäßen Nucleinsäuresequenzen ein Polypeptid mit den Eigenschaften einer Fructosyltransferase aus Pilzen, besonders bevorzugt aus *Aspergillus* und ganz besonders bevorzugt einer Fructosyltransferase aus *Aspergillus sydowi.*

Bei den erfindungsgemäßen Nucleinsäuremolekülen kann es sich sowohl um DNAals auch RNA-Moleküle handeln. Entsprechende DNA-Moleküle sind beispielsweise genomische DNA- oder cDNA-Moleküle. Die erfindungsgemäßen Nucleinsäuremoleküle können aus natürlichen Quellen isoliert werden, vorzugsweise aus Pilzen, insbesondere *Aspergillus* und bevorzugt aus *Aspergillus sydowi,* oder sie können nach bekannten Verfahren synthetisiert werden.
Mittels gängiger molekularbiologischer Techniken ist es ferner möglich (siehe z.B. Sambrook et al., 1989, Molecular Cloning, A Laboratory Manual, 2. Aufl. Cold Spring Harbor Laboratory Press, Cold Spring Harbor, NY) verschiedenartige Mutationen in die erfindungsgemäßen Nucleinsäuremoleküle einzuführen, wodurch es zur Synthese von Proteinen mit eventuell veränderten biologischen Eigenschaften kommt. Hierbei ist zum einen die Erzeugung von Deletionsmutanten möglich, bei denen durch fortschreitende Deletionen vom 5'- oder vom 3'- Ende der codierenden DNA-Sequenz Nucleinsäuremoleküle erzeugt werden, die zur Synthese entsprechend verkürzter Proteine führen. Andererseits können auch gezielt Enzyme hergestellt werden, die aufgrund der Addition von Signalsequenzen in verschiedenen Kompartimenten lokalisiert sind. Um eine Lokalisierung der erfindungsgemäßen Proteine im Cytosol zu erreichen, müssen der unter SEQ ID No. 2 angegebenen Sequenz keine Signalsequenzen hinzugefügt werden.

Ferner ist auch die Einführung von Punktmutationen denkbar an Positionen, bei denen eine Veränderung der Aminosäuresequenz einen Einfluß beispielweise auf die Enzymaktivität oder die Regulierung des Enzyms hat. Auf diese Weise können z.B. Mutanten hergestellt werden, die einen veränderten Kₘ-Wert besitzen oder nicht mehr den normalerweise in der Zelle vorliegenden Regulationsmechanismen z.B. über allosterische Regulation oder kovalente Modifizierung unterliegen.
Des weiteren können Mutanten hergestellt werden, die eine veränderte Substrat- oder Produktspezifität aufweisen. Weiterhin können Mutanten hergestellt werden, die ein verändertes Aktivitäts-Temperatur-Profil aufweisen.
Für die gentechnische Manipulation in prokaryontischen Zellen können die erfindungsgemäßen Nucleinsäuremoleküle oder Teile dieser Moleküle in Plasmide eingebracht werden, die eine Mutagenese oder eine Sequenzveränderung durch Rekombination von DNA-Sequenzen erlauben. Mit Hilfe von Standardverfahren (vgl. Sambrook et al., 1989, Molecular Cloning: A Laboratory Manual, 2. Aufl., Cold Spring Harbor Laboratory Press, NY, USA) können Basenaustausche vorgenommen oder natürliche oder synthetische Sequenzen hinzugefügt werden. Für die Verbindung der DNA-Fragmente untereinander können an die Fragmente Adaptoren oder Linker angesetzt werden. Ferner können Manipulationen, die passende Restriktionsschnittstellen zur Verfügung stellen oder die überflüssige DNA oder Restriktionsschnittstellen entfernen, eingesetzt werden. Wo Insertionen, Deletionen oder Substitutionen in Frage kommen, können *in vitro*-Mutagenese, "primer repair", Restriktion oder Ligation verwendet werden. Als Analysemethode werden im allgemeinen eine Sequenzanalyse, eine Restriktionsanalyse und weitere biochemisch-molekularbiologische Methoden durchgeführt.

Die Erfindung betrifft ferner Vektoren, die erfindungsgemäße Nucleinsäuremoleküle enthalten. Vorzugsweise handelt es sich dabei um Plasmide, Cosmide, Viren, Bacteriophagen und andere in der Gentechnik übliche Vektoren.

Vorzugsweise ist das erfindungsgemäße Nucleinsäuremolekül im erfindungsgemäßen Vektor mit regulatorischen Elementen operativ verbunden, die die Transkription und Synthese einer translatierbaren RNA in pro- und/oder eukaryontischen Zellen gewährleisten. Beispielsweise enthält ein erfindungsgemäßer Vektor die folgenden Elemente:
1. Einen Promotor, der die Transkription nachgeschalteter Codierregionen in Zellen des Wirtsorganismus sicherstellt, und gegebenenfalls Enhancer-Elemente.
2. Eine Codierregion als Fusion an den Promotor, die zumindest ein offenes Leseraster für die Translation eines Polypeptids enthält. Erfindungsgemäß handelt es sich bei der Codierregion um ein erfindungsgemäßes Nucleinsäuremolekül.
3. Gegebenenfalls weitere Sequenzen als Fusion an die Codierregion, beispielsweise Transkriptionsterminationssignale, wenn dies zur erfolgreichen Genexpression in einem bestimmten Wirtsorganismus erforderlich ist, oder Signalsequenzen, die die subzelluläre Lokalisation des Genprodukts beeinflussen oder die die Sekretion des Proteins bewirken.

Ein derartiger Vektor kann weitere Gene enthalten, wie Markergene, die die Selektion des Vektors in einer geeigneten Wirtszelle und unter geeigneten Bedingungen erlauben. Die Expression des erfindungsgemäßen Nucleinsäuremoleküls umfaßt die Transkription des Nucleinsäuremoleküls in translatierbare mRNA. Regulatorische Elemente, die die Expression des Nucleinsäuremolküls in prokaryontischen oder eukaryontischen Zellen gewährleisten, sind dem Fachmann bekannt. Mögliche regulatorische Elemente, die für die Expression des erfindungsgemäßen Nucleinsäuremoleküls in prokaryontischen Wirtszellen geeignet sind, umfassen beispielsweise den P_{L}, lac, trp oder tac Promotor in *E*. *coli.* Besonders bevorzugt wird der in *E*. *coli* durch IPTG induzierbare lacZ-Promoter verwendet. Beispiele für regulatorische Elemente, die die Expression in eukaryontischen Wirtszellen erlauben, sind der AOX1 und der GAL1 Promotor in Hefe oder der CMV-, SV40-, RSV-Promotor, CMV-Enhancer, SV40-Enhancer oder ein Globin-Intron in Säugetierzellen oder anderen tierischen Zellen. Für die Expression in Hefe wird bevorzugt der in Hefe hochaktive Promotor des Alkohol-Dehydrogenase-Gens aus *Saccharomyces cerevisiae* verwendet. Weitere geeignete Vektorsysteme sind im Stand der Technik beschrieben, beispielsweise Sambrook, Molecular Cloning A Laboratory Manual (1989), Cold Spring Harbor Laboratory Press N.Y., und in Ausubel, Current Protocols in Molecular Biology (1989), Green Publishing Associates and Wiley Interscience, N.Y..

Regulatorische Elemente für die Expression der erfindungsgemäßen Nucleinsäuremoleküle in Pflanzenzellen sind prinzipiell jeder in pflanzlichen Zellen aktive Promoter, Enhancer, Terminator, etc. Der Promotor kann dabei so gewählt sein, daß die Expression in den erfindungsgemäßen Pflanzen konstitutiv erfolgt oder nur in einem bestimmten Gewebe zu einem bestimmten Zeitpunkt der Pflanzenentwicklung oder zu einem durch äußere Einflüsse determinierten Zeitpunkt. In Bezug auf die Pflanze kann der Promotor homolog oder heterolog sein.
Sinnvolle Promotoren sind z.B. der Promotor der 35S RNA des Cauliflower Mosaic Virus (siehe beispielsweise US 5,352,605) und der Ubiquitin-Promotor (siehe beispielsweise US 5,614,399) für eine konstitutive Expression, der Patatingen-Promotor B33 (Rocha-Sosa, EMBO J. 8 (1989), 23-29) für eine knollenspezifische Expression in Kartoffeln oder ein Promotor, der eine Expression lediglich in photosynthetisch aktiven Geweben sicherstellt, z.B. der ST-LS1-Promotor (Stockhaus, Proc. Natl. Acad. Sci. USA 84 (1987), 7943-7947; Stockhaus, EMBO J. 8 (1989), 2445-2451), der Ca/b-Promotor (siehe beispielsweise US 5,656,496, US 5,639,952, Bansal, Proc. Natl. Acad. Sci. USA 89 (1992), 3654-3658) und der Rubisco SSU-Promotor (siehe beispielsweise US 5,034,322, US 4,962,028). Es können jedoch auch Promotoren verwendet werden, die nur zu einem durch äußere Einflüsse determinierten Zeitpunkt aktiviert werden (siehe beispielsweise WO 93107279). Von besonderem Interesse können hierbei Promotoren von heatshock Proteinen sein, die eine einfache Induktion erlauben. Ferner können samenspezifische Promotoren, wie z.B. der USP-Promotor aus *Vicia faba,* der eine samenspezfische Expression in *Vicia faba* und anderen Pflanzen gewährleistet, verwendet werden (Fiedler, Plant Mol. Biol. 22 (1993), 669-679; Bäumlein, Mol. Gen. Genet. 225 (1991), 459-467). Ferner können auch fruchtspezifische Promotoren eingesetzt werden, wie z.B. in WO 91/01373 beschrieben. Für die Expression in reifenden Tomatenfrüchten eignen sich z.B. cis-regulatorische Elemente eines Polygalacturonase-Promotors aus Tomate, die im äußeren bzw. inneren Pericarp aktiv sind (Nicholass et al., Plant Mol. Biol. 28 (1995), 423-435; Montgomery et al., Plant Cell 5 (1993), 1049-1062). Einen weiteren fruchtspezifischen Promotor für die Tomate beschreiben Van Haaren et al. (Plant Mol. Biol. 21 (1993), 625-640).
Darüberhinaus können Promotoren für eine endospermspezifische Expression verwendet werden, wie z.B. der Glutelinpromotor (Leisy, Plant Mol. Biol. 14 (1990), 41-50; Zheng, Plant J. 4 (1993), 357-366), der HMG-Promotor aus Weizen, der USP-Promotor, der Phaseolinpromotor oder Promotoren von Zein-Genen aus Mais (Pedersen, Cell 29 (1982), 1015-1026; Quattrocchio, Plant Mol. Biol. 15 (1990), 81-93) oder der shrunken-1-Promotor (sh-1) aus Mais (Werr, EMBO J. 4 (1985), 1373-1380).
Die Expression der erfindungsgemäßen Nucleinsäuremoleküle ist insbesondere in solchen Organen der Pflanze von Vorteil, die einen erhöhten Gehalt an Saccharose aufweisen oder Saccharose speichern. Solche Organe sind z.B. die Rübe der Zuckerrübe oder der Stamm des Zuckerrohrs und der Zuckerhirse. Besonders bevorzugt werden daher Promotoren verwendet, die die Expression in diesen Organen vermitteln, wie beispielsweise der Patatin-Promotor B33 aus *Solanum tuberosum.* Zur spezifischen Expression im Stamm von Zuckerrohrpflanzen kann der Ubiquitin-Promotor in Kombination mit dem ersten Intron verwendet werden.
Die erfindungsgemäßen Vektoren können darüberhinaus weitere Funktionseinheiten besitzen, die eine Stabilisierung des Vektors in einem Wirtsorganismus bewirken, wie einen bakteriellen Replikationsursprung oder die 2-Mikron-DNA zur Stabilisierung und autonomen Replikation in Hefe. Ferner können "left border"- und "right border"-Sequenzen agrobakterieller T-DNA enthalten sein, wodurch eine stabile Integration in das Erbgut von Pflanzen ermöglicht wird.
Ferner kann eine Terminationssequenz vorhanden sein, die der korrekten Beendigung der Transkription dient sowie der Addition eines Poly-A-Schwanzes an das Transkript, dem eine Funktion bei der Stabilisierung der Transkripte beigemessen wird. Derartige Elemente sind in der Literatur beschrieben (vgl. z.B. Gielen, EMBO J. 8 (1989), 23-29) und sind beliebig austauschbar.

In einer bevorzugten Ausführungsform umfaßt das in dem erfindungsgemäßen Vektor enthaltene Nucleinsäuremolekül eine Region, die eine funktionale Signalsequenz zur Sekretion des codierten Enzyms enthält. Derartige Sequenzen sind bekannt.
Ein Signalpeptid, das die Lokalisation des Proteins in der Vakuole gewährleistet, ist beispielsweise das Signalpeptid der Carboxypeptidase Y aus Hefe (CPY). Das entsprechende Gen ist beispielsweise in Valls et al. (Cell 48, 887-899) beschrieben. Pflanzliche Signalsequenzen sind z.B. die der Lektingene aus Gerste (Raikhel und Lemer, Dev. Genet. 12 (1991), 255-269) oder die 43 Aminosäuren aus dem N-terminalen Bereich des reifen Phytohämagglutinins der Bohne (Tague et al., Plant Cell 2 (1990), 533-546). Ein Beispiel für ein C-terminales Signalpeptid ist das der Chitinase (Neuhaus et al., Plant J. 5 (1994), 45-54).
Eine bevorzugte Signalsequenz ist beispielsweise die Signalsequenz des Proteinase-Inhibitor II Gens aus Kartoffel. Es kann aber auch jede andere Signalsequenz, die zur Sekretion eines Polypeptids in dem gewählten Wirt führt, verwendet werden. Die sektretierte Fructosyltransferase kann aus dem Kulturmedium gewonnen und für *in vitro* Synthesen eingesetzt werden.

In einer besonders bevorzugten Ausführungsform enthält das in dem Vektor enthaltene Nucleinsäuremolekül eine Region, die eine Signalsequenz zur vakuolären Lokalisation in pflanzlichen Zellen codiert, bevorzugt die aus dem Patatingen der Kartoffel (Sonnewald, Plant J. 1 (1998), 95-106). Dies erlaubt die subzelluläre Lokalisation der Fructosyltransferase in den Vakuolen von gentechnisch veränderten Pflanzenzellen und Pflanzen, beispielsweise Zuckerrübe oder Kartoffel, und die Akkumulation von hochmolekularen Polyfructosen des Inulintyps in den Vakuolen. Weitere vakuoläre Signalsequenzen sind beispielsweise beschrieben bei Matsuoka (Journal of Experimental Botany 50 (1999), 165-174), Chrispeels (Cell 68 (1992), 613-616), Matsuoka (Proc. Natl. Acad. Sci. USA 88 (1991), 834-838), Bednarek (Plant Cell 3 (1991), 1195-1206), Nakamura (Plant Phys. 101 (1993) 1-5).

In einer weiteren Ausführungsform der Erfindung enthält das in dem Vektor enthaltene Nucleinsäuremolekül eine Region, die eine Signalsequenz zur plastidären Lokalisation in pflanzlichen Zellen codiert.
Als Signalsequenz kann beispielsweise die Signalsequenz der Ferrodoxin: NADP(+)-oxidoreductase (FNR) aus Spinat verwendet werden. Die Sequenz enthält den 5'-nichttranslatierten Bereich sowie die flankierende Transitpeptidsequenz der cDNA des plastidären Proteins Ferrodoxin:NADP(+)-oxidoreductase aus Spinat (Nucleotid -171 bis +165; Jansen, Current Genetics 13 (1988), 517-522).
Ferner kann als Signalsequenz beispielsweise das Transitpeptid des waxy-Proteins aus Mais plus die ersten 34 Aminosäuren des maturen waxy-Proteins (Klösgen, Mol. Gen. Genet. 217 (1989), 155-161) verwendet werden. In einer bevorzugten Ausführungsform der Erfindung wird das Transitpeptid des waxy-Proteins aus Mais verwendet ohne die ersten 34 Aminosäuren des maturen waxy-Proteins.

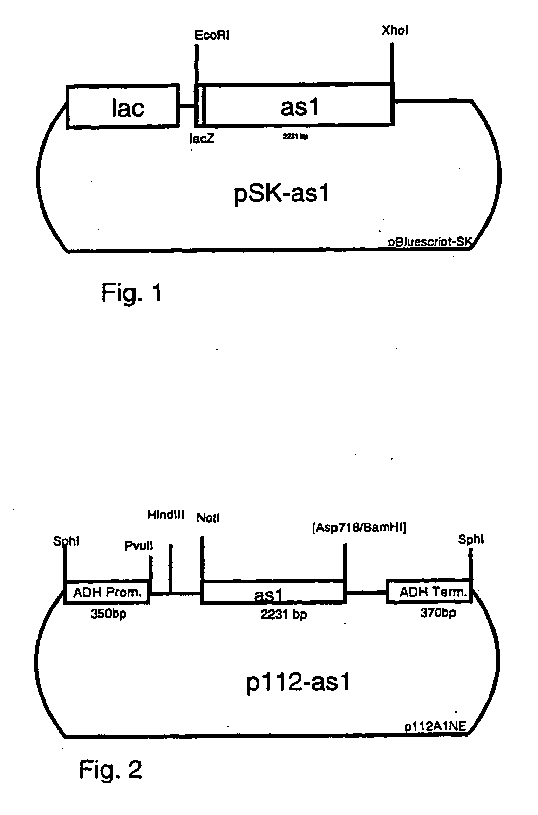

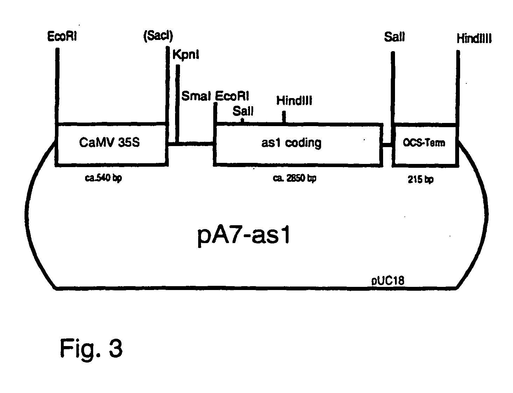

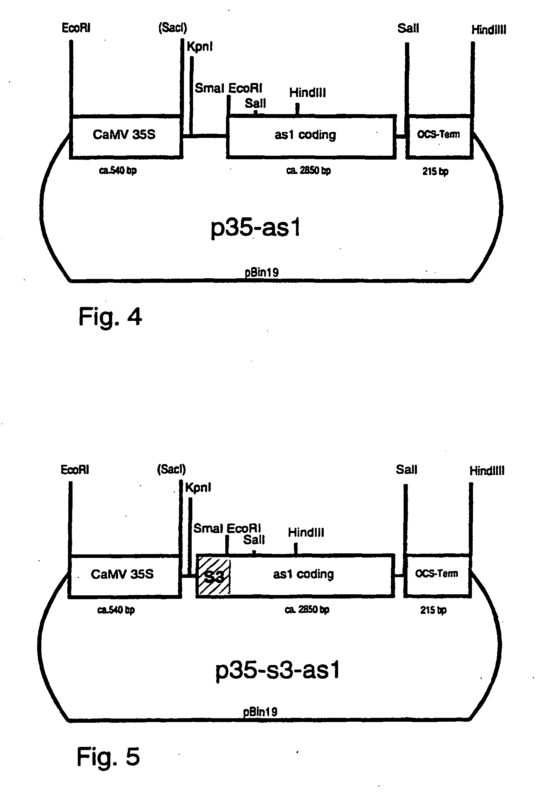

In einer besonders bevorzugten Ausführungsform betrifft die Erfindung die Plasmide pSK-as1, p112-as1, pA7-as1, p35-as1, p35-s3-as1, deren Konstruktion in den Beispielen beschrieben ist (Fig. 1 bis 5).

Die erfindungsgemäßen Nucleinsäuremoleküle und Expressionsvektoren gestatten die Herstellung von Polyfructosen des Inulintyps in verschiedenen Wirtsorganismen, insbesondere Pflanzen, Pilzen und Bakterien. Die codierten Enzyme können auch außerhalb der Wirtsorganismen zur Produktion von Polyfructosen des Inulintyps eingesetzt werden. D.h. es ist insbesondere möglich, die erfindungsgemäßen Nucleinsäuremoleküle zur Produktion der durch sie codierten Proteine in beliebigen Wirtszellen zu verwenden, das Protein aus den Wirtszellen und/oder dem Kulturmedium zu gewinnen und zur in-vitro Synthese von Inulin einzusetzen.
So kann beispielsweise ein Konstrukt, das den Alkoholdehydrogenase-Promotor und ein erfindungsgemäßes Nucleinsäuremolekül enthält, zur Transformation von *Saccharomyces cerevisiae* eingesetzt werden. Da Hefen nicht in der Lage sind, Saccharose aufzunehmen, sollten Zellen verwendet werden, die aufgrund der Einführung einer heterologen DNA-Sequenz einen Saccharosetransporter exprimieren. Die Herstellung solcher Zellen ist z.B. beschrieben in Riesmeier et al. (EMBO J. 11 (1992), 4705-4713). Mit dem oben beschriebenen Konstrukt transformierte Hefen bilden langkettige Polyfructosen vom Inulintyp. Da die Fructosyltransferase von *Aspergillus sydowi* kein Signalpeptid besitzt, wird sie nicht sekretiert. Die langkettigen Polyfructosen entstehen daher in den Hefezellen. Hefezellen, die diese Polyfructosen enthalten, können direkt als Nahrungsmittelzusätze eingesetzt werden. Sollen die Polyfructosen fermentativ im Kulturmedium gewonnen werden, kann eine Signalsequenz zur Sekretion an ein erfindungsgemäßes Nucleinsäuremolekül fusioniert werden.

In einer weiteren Ausführungsform betrifft die Erfindung Wirtszellen, die die erfindungsgemäßen Nucleinsäuremoleküle oder Vektoren transient oder stabil enthalten, wobei die Nucleinsäuremoleküle in Bezug auf die Wirtszelle heterolog sind. Unter einer Wirtszelle wird ein Organismus verstanden, der in der Lage ist, *in vitro* rekombinierte DNA aufzunehmen und gegebenenfalls die von den erfindungsgemäßen Nucleinsäuremolekülen codierten Proteine zu synthetisieren. Die Wirtszellen können entweder prokaryontischen oder eukaryontischen Ursprung sein. Der Begriff "prokaryontisch" schließt dabei alle Bakterien ein, die mit einem erfindungsgemäßen Nucleinsäuremolekül transformiert oder transfiziert werden können und vorteilhafterweise die Expression eines Proteins mit Fructosyltransferaseaktivität erlauben. Prokaryontische Wirtszellen schließen beispielsweise gram-negative wie auch gram-positive Bakterien ein, wie z.B. *E. coli, S. typhimurium, Serratia marcescens* und *Bacillus subtilis.* Der Begriff "eukaryontisch" schließt Insektenzellen, pilzliche Zellen, Pflanzenzellen, tierische und menschliche Zellen ein. Bevorzugte pilzliche Zellen sind beispielsweise solche, die für die Fermentation benutzt werden oder benutzt werden können, insbesondere *Saccharomyces,* dabei besonders bevorzugt *S. cerevisiae, Schizosaccharomyces, Kluyveromyces, Pichia* etc. Bevorzugt ist eine solche pilzliche Wirtszelle eine Zelle aus der Gattung *Aspergillus* und besonders bevorzugt aus der Spezies *Aspergillus niger.* Interessant ist insbesondere die Expression der erfindungsgemäßen Fructosyltransferase in diesen Zellen in Kombination mit einer sekretorischen Signalsequenz, z.B. der aus dem Patatingen oder des 1-SST-Gens aus *Aspergillus foetidus* (Rehm et al., J. Bac. 180 (1998), 1305-1319). Dadurch wird die Fructosyltransferase in das Medium sekretiert. Vorteilhafterweise werden Zellen verwendet, die eine reduzierte oder gar keine sekretorische Invertaseaktivität besitzen. Pilzspezies, die keine eigene Invertaseaktivität besitzen, sind z.B. *Trichoderma reesei.* Ein Protokoll zur Expression einer β-Fructofuranosidase (der Invertase aus *A. niger*) ist beispielsweise beschrieben in Bergès et al. (Curr. Genet. 24 (1993), 53-59). Ein erfindungsgemäßes Nucleinsäuremolekül, das ein Protein mit Fructosyltransferaseaktivität codiert, oder ein entsprechender Vektor kann durch dem Fachmann geläufige Techniken in die Wirtszelle transfiziert oder transformiert werden. Verfahren zur Herstellung von fusionierten, operativ verknüpften Genen und deren Expression in geeigneten Wirtszellen sind dem Fachmann wohlbekannt und beschrieben beispielsweise in Sambrook oder Ausubel, siehe oben. Bevorzugte Wirte sind *E. coli,* Pilze, insbesondere Hefen, und Pflanzenzellen.

Bei der Expression der erfindungsgemäßen Nucleinsäuremoleküle in Pflanzen besteht grundsätzlich die Möglichkeit, daß das synthetisierte Protein in jedem beliebigen Kompartiment der pflanzlichen Zelle lokalisiert sein kann. Um die Lokalisation in einem bestimmten Kompartiment zu erreichen, kann das erfindungsgemäße Nucleinsäuremolekül mit DNA-Sequenzen verknüpft werden, die die Lokalisierung in dem jeweiligen Kompartiment gewährleisten; siehe oben. Derartige Sequenzen sind bekannt (siehe beispielsweise Braun, EMBO J. 11 (1992), 3219-3227; Wolter, Proc. Natl. Acad. Sci. USA 85 (1988), 846-850; Sonnewald, Plant J. 1 (1991), 95-106; Rocha-Sosa, EMBO J. 8 (1989), 23-29). Die erfindungsgemäßen Wirte umfassen somit auch transgene Pflanzenzellen, Pflanzengewebe und Pflanzen, die mit einem oder mehreren erfindungsgemäßen Nucleinsäuremolekül(en) transformiert wurden, sowie transgene Pflanzenzellen, die von derartig transformierten Zellen abstammen. Derartige Zellen enthalten ein oder mehrere erfindungsgemäße(s) Nucleinsäuremolekül(e), wobei diese(s) vorzugsweise mit regulatorischen DNA-Elementen verknüpft ist/sind, die die Transkription in pflanzlichen Zellen gewährleisten, insbesondere mit einem Promotor. Derartige Zellen lassen sich von natürlicherweise vorkommenden Pflanzenzellen dadurch unterscheiden, daß sie mindestens ein erfindungsgemäßes Nucleinsäuremolekül enthalten, das natürlicherweise in diesen Zellen nicht vorkommt.
In einer weiteren Ausführungsform betrifft die vorliegende Erfindung Pflanzenzellen, die das erfindungsgemäße Protein im Cytosol enthalten. Hierzu ist die als SEQ ID No. 2 angegebene Sequenz ohne weitere Signalsequenzen zu verwenden.

In einer weiteren bevorzugten Ausführungsform betrifft die vorliegende Erfindung Pflanzenzellen, die das erfindungsgemäße Protein in den Plastiden enthalten.
Zur Erzielung einer plastidären Lokalisation des erfindungsgemäßen Proteins kann man die erfindungsgemäßen Nucleinsäuremoleküle und/oder die erfindungsgemäßen Vektoren in der oben beschriebenen Weise modifizieren.

Da die Vakuole in der Regel große Mengen an Saccharose speichern kann, die dem erfindungsgemäßen Protein als Substrat dient, ist dieses Kompartiment gut geeignet, um Pflanzenzellen zu erzeugen, die aufgrund der Aktivität eines erfindungsgemäßen Proteins Polyfructose in den Vakuolen produzieren.
In einer besonders bevorzugten Ausführungsform betrifft die vorliegende Erfindung daher Pflanzenzellen, die das erfindungsgemäße Protein in der Vakuole enthalten. Weiter oben wurde bereits erläutert, wie die erfindungsgemäßen Nucleinsäuremoleküle und/oder Vektoren konstruiert sein müssen, um eine vakuoläre Lokalisation des erfindungsgemäßen Proteins zu vermitteln.

Die transgenen Pflanzenzellen und Pflanzengewebe können nach dem Fachmann bekannten Techniken zu ganzen Pflanzen regeneriert werden. Die durch Regeneration der erfindungsgemäßen transgenen Pflanzenzellen erhältlichen Pflanzen, die solche Pflanzenzellen enthalten, sind ebenfalls Gegenstand der vorliegenden Erfindung. Ferner sind Gegenstand der Erfindung Pflanzen, die die oben beschriebenen transgenen Pflanzenzellen enthalten. Die erfindungsgemäßen Pflanzenzellen können zu jeder beliebigen Pflanzenspezies gehören, vorzugsweise zu monokotyledonen oder dikotyledonen Pflanzen. Bevorzugt handelt es sich um Pflanzenzellen aus landwirtschaftlichen Nutzpflanzen, d.h. aus Pflanzen, die vom Menschen kultiviert werden für Zwecke der Ernährung oder für technische, insbesondere industrielle Zwecke. Vorzugweise betrifft die Erfindung faserbildende (z.B. Flachs, Hanf, Baumwolle), ölspeichernde (z.B. Raps, Sonnenblume, Sojabohne), zuckerspeichernde (z.B. Zuckerrübe, Zuckerrohr, Zuckerhirse, Banane) und proteinspeichernde Pflanzen (z.B. Leguminosen).
In einer weiteren bevorzugten Ausführungsform betrifft die Erfindung Futterpflanzen (z.B. Futter- und Weidegräser, Alfalfa, Klee etc.), Gemüsepflanzen (z.B. Melone, Tomate, Banane, Chicoree, Lauch, Spargel, Mohrrübe) oder stärkespeichernde Pflanzen (Weizen, Gerste, Hafer, Roggen, Kartoffel, Mais, Reis, Erbse, Maniok, Mungbohne).
In einer weiteren bevorzugten Ausführungsform betrifft die Erfindung Pflanzenzellen aus Saccharose enthaltenden Pflanzen (z.B. Zuckerrübe, Kartoffel, Reis, Weizen, Zuckerrohr, etc.). Besonders bevorzugt sind Zuckerrübe, Chicoree, Reis, Mais, Kartoffel, Zuckerrohr und Weizen. Die Erfindung umfaßt ebenfalls Vermehrungsmaterial und Ernteprodukte der erfindungsgemäßen Pflanzen, beispielsweise Früchte, Samen, Knollen, Wurzelstöcke, Sämlinge, Stecklinge, Calli, Zellkulturen etc.

Die vorliegende Erfindung betrifft auch Verfahren zur Herstellung transgener Pflanzen, wobei
(a) eine pflanzliche Zelle genetisch modifiziert wird durch die Einführung eines erfindungsgemäßen Nucleinsäuremoleküls und/oder eines erfindungsgemäßen Vektors, wobei das Nucleinsäuremolekül in Bezug auf die Zelle heterolog ist; und
(b) aus der Zelle eine Pflanze regeneriert wird; und gegebenenfalls
(c) aus der Pflanze nach (b) weitere Pflanzen erzeugt werden.

Der Begriff "genetisch modifiziert" bedeutet dabei im Zusammenhang mit der vorliegenden Erfindung, daß die pflanzliche Zelle durch Einführung eines erfindungsgemäßen Nucleinsäuremoleküls in ihrer genetischen Information verändert ist, und daß das Vorhandensein oder die Expression des erfindungsgemäßen Nucleinsäuremoleküls zu einer phänotypischen Veränderung führt. Phänotypische Veränderung bedeutet dabei vorzugsweise eine meßbare Veränderung einer oder mehrerer Funktionen der Zellen. Beispielsweise zeigen genetisch modifizierte erfindungsgemäße Pflanzen eine Aktivität des erfindungsgemäßen Proteins oder eine erhöhte Fructosyltransferase-Gesamtaktivität.
Die Regeneration von Pflanzen gemäß Schritt (b) kann nach dem Fachman bekannten Methoden erfolgen.
Die Erzeugung weiterer Pflanzen gemäß Schritt (c) der erfindungsgemäßen Verfahren kann z.B. erfolgen durch vegetative Vermehrung (beispielsweise über Stecklinge, Knollen oder über Calluskultur und Regeneration ganzer Pflanzen) oder durch generative Vermehrung. Die generative Vermehrung findet dabei vorzugsweise kontrolliert statt, d.h. es werden ausgewählte Pflanzen mit bestimmten Eigenschaften miteinander gekreuzt und vermehrt.
Die vorliegende Erfindung betrifft auch die durch die erfindungsgemäßen Verfahren erhältlichen Pflanzen, sofern diese erfindungsgemäße Pflanzenzellen enthalten.

Die vorliegende Erfindung betrifft auch Vermehrungsmaterial erfindungsgemäßer Pflanzen, wobei das Vermehrungsmaterial erfindungsgemäße Pflanzenzellen enthält. Der Begriff Vermehrungsmaterial umfaßt dabei jene Bestandteile der Pflanze, die geeignet sind zur Erzeugung von Nachkommen auf vegetativem oder generativem Weg. Für die vegetative Vermehrung eignen sich beispielsweise Früchte, Samen, Sämlinge, Protoplasten, Zellkulturen etc. Vorzugsweise handelt es sich bei dem Vermehrungsmaterial um Knollen und Samen.

In einer weiteren Ausführungsform betrifft die vorliegende Erfindung erntebare Pflanzenteile erfindungsgemäßer Pflanzen, wie Früchte, Blätter, Speicherwurzeln, Wurzeln, Blüten, Knospen, Sprosse oder Stämme, vorzugsweise Samen oder Knollen, wobei die Pflanzenteile erfindungsgemäße Pflanzenzellen enthalten.

In einer weiteren Ausführungsform betrifft die vorliegende Erfindung Futtermittel und/oder Nahrungsmittel, die die erfindungsgemäßen erntebaren Pflanzenteile, bevorzugt Samen oder Knollen, enthalten.

Vorzugsweise wirken die erfindungsgemäßen Pflanzenteile nach Verzehr im Vergleich zu entsprechenden Pflanzenteilen von Pflanzen, die nicht in der erfindungsgemäßen Weise genetisch modifiziert worden sind, vorteilhaft auf die Gesundheit des Menschen und/oder des Tieres. Entsprechendes gilt für die erfindungsgemäßen Futtermittel und/oder Nahrungsmittel. Bei Menschen beispielsweise kann der Verzehr der erfindungsgemäßen Nahrungsmittel zu einer verbesserten Zusammensetzung der Darmflora, insbesondere zu einer Steigerung des Gehalts an Bifidobakterien im Darm führen, was sich vermutlich positiv auf die Gesundheit des Menschen auswirkt (Izzo, Trends in Food Science & Technology 9 (1998), 255-257). Bei diesen positiven Effekten handelt es sich vorzugsweise um prophylaktische oder die Nahrungsverwertung unterstützende Effekte.

Gegenstand der Erfindung sind auch Verfahren zur Herstellung erfindungsgemäßer Wirtszellen, wobei geeignete Wirtszellen mit einem erfindungsgemäßen Nucleinsäuremolekül oder Vektor transformiert werden. Verfahren für die Transformation der verschiedenen in Betracht kommenden Wirtszellen sind dem Fachmann geläufig.

In einer weiteren Ausführungsform betrifft die vorliegende Erfindung Verfahren zur Herstellung einer Fructosyltransferase, bei dem ein erfindungsgemäßer Wirt unter Bedingungen kultiviert wird, die für die Expression des erfindungsgemäßen Nucleinsäuremoleküls genügen und anschließend die Fructosyltransferase aus der Kultur, d.h. den Zellen und/oder dem möglicherweise vorhandenen Kulturmedium, isoliert wird. In dem vorgenannten Verfahren werden die transformierten oder transfizierten Wirtszellen beispielsweise in Fermentern kultiviert, bis eine optimale Zelldichte erreicht ist. Gegebenenfalls kann bei induzierbaren Promotoren erst am Schluß der Fermentation die Expression des Proteins, das durch das erfindungsgemäße Nucleinsäuremolekül codiert wird, induziert werden. Das so exprimierte Protein kann dann anhand von bekannten Techniken aus dem Medium, Zelllysaten, oder zellulären Membranfraktionen nach bekannten Techniken gereinigt werde. Die Isolation und Reinigung der beispielsweise mikrobiell exprimierten Proteine kann durch präparative chromatographische oder auch immunologische Trenntechniken erreicht werden, beispielsweise unter Zuhilfenahme von monoclonalen oder polyclonalen Antikörpern, die das von dem erfindungsgemäßen Nucleinsäuremolekül codierte Protein erkennen. In diesem Zusammenhang mag erwähnt werden, daß das von dem erfindungsgemäßen Nucleinsäuremolekül codierte Protein mit Fructosyltransferaseaktivität auch weitere funktionelle Aminosäuresequenzen enthalten kann, beispielsweise Protein tags (GST, GFP, Flag, HA-Peptid, His-tag), die von heterologen Proteinen stammen können oder synthetisch erzeugt wurden.

Die Erfindung betrifft ferner Proteine, die Fructosyltransferaseaktivität besitzen, d.h. Fructosyltransferasen, die durch die erfindungsgemäßen Nucleinsäuremoleküle codiert werden oder durch das erfindungsgemäße Verfahren erhältlich sind. Die erfindungsgemäßen Fructosyltransferasen können vorzugsweise zur Herstellung von Polyfructosen des Inulintyps verwendet werden. Andererseits können sie auch zur Herstellung von Antikörpern dienen, die wiederum zum Nachweis und/oder zur Reinigung von Fructosyltransferasen genutzt werden können.

Gegenstand der Erfindung sind auch Nucleinsäuremoleküle, die eine Früctosyltransferase codieren und die spezifisch mit erfindungsgemäßen Nucleinsäuremolekülen oder Teilen davon hybridisieren, wobei die Hybridisierung unter folgenden Bedingungen erfolgt: Hybridisierung in 50% Formamid, 5 x SSC, 5 x Denhardt's Lösung, 40 mM Natriumphosphat pH 6,8; 0,5% (Gew./Vol.) BSA, 1% (Gew./Vol.) SDS, 0,1 mg/ml Heringsperma-DNA bei einer Hybridisierungstemperatur von 42°C und anschließendes Waschen der Filter in 0,5 x SSC/0,5% SDS bei 60°C. Dabei handelt es sich vorzugsweise um Oligonucleotide mit einer Länge von mindestens 10, insbesondere von mindestens 15 und besonders bevorzugt von mindestens 50 Nucleotiden. Die erfindungsgemäßen Oligonucleotide können beispielsweise als Primer für eine PCR-Reaktion, als Hybridisierungssonden oder ähnliches verwendet werden.

Gegenstand der vorliegenden Erfindung sind auch Verfahren zur Herstellung von Polyfructose, insbesondere des Inulintyps, wobei erfindungsgemäße Wirtszellen, oder diese enthaltende Wirtsorganismen, unter Bedingungen kultiviert werden, die die Expression der erfindungsgemäßen Fructosyltransferase sowie die Synthese von Polyfructose erlauben.
Durch die Bereitstellung der erfindungsgemäßen Nucleinsäuremoleküle ist es möglich, mit Hilfe gentechnischer Methoden Polyfructosen, insbesondere solche des Inulintyps, in Organismen herzustellen, wie es bisher durch konventionelle Verfahren nicht möglich war. Möglich ist somit die Expression der erfindungsgemäßen Nucleinsäuremoleküle in Wirten wie Bakterien, Pilzen oder pflanzlichen Zellen, um die Aktivität der entsprechenden Fructosyltransferase zu erhöhen, oder die Einführung in Zellen, die dieses Enzym normalerweise nicht exprimieren. Die erfindungsgemäßen Wirtszellen synthetisieren aufgrund der Expression bzw. zusätzlichen Expression mindestens eines erfindungsgemäßen Nucleinsäuremoleküls Polyfructose, insbesondere solche des Inulintyps. Gegenstand der vorliegenden Erfindung sind somit auch die aus den erfindungsgemäßen Wirtszellen sowie dem Vermehrungsmaterial und bei Pflanzen aus den Pflanzen oder aus deren Emteprodukten erhältlichen Polyfructosen, insbesondere solche des Inulintyps.
Somit betrifft die vorliegende Erfindung insbesondere Verfahren zur Herstellung von Polyfructosen, insbesondere solche des Inulintyps, umfassend:
(a) Kultivierung einer erfindungsgemäßen Wirtszelle, oder eines eine derartige Zelle enthaltenden Wirts, unter Bedingungen, die die Produktion von Fructosyltransferase und Umsetzung von gegebenenfalls von außen zugeführter Saccharose, oder eines äquivalenten Substrats zu Polyfructosen des Inulintyps erlauben; und
(b) Gewinnung der so hergestellten Polyfructose aus den kultivierten Wirtszellen, Wirten oder aus dem Medium.

Bei den Wirtszellen handelt es sich vorzugsweise um Pflanzenzellen und bei den Wirten vorzugsweise um Pflanzen. Eine Methode zur Gewinnung von Polyfructose, insbesondere solche des Inulintyps, aus Pflanzen beschreibt z.B. Vogel (in: Inulin and Inulin-containing Crops, Elsevier Science Publishers B.V. Amsterdam, A. Fuchs (Ed.) (1993), 65-75).

Gegenstand der vorliegenden Erfindung ist auch ein in-vitro Verfahren zur Herstellung von Polyfructose, insbesondere solche des Inulintyps, umfassend:
(a) Inkontaktbringen von Saccharose oder eines äquivalenten Substrats mit einer erfindungsgemäßen Fructosyltransferase unter Bedingungen, die die Umsetzung zu Polyfructose erlauben; und
(b) Gewinnung der so hergestellten Polyfructose.

Ein zu Saccharose äquivalentes Substrat ist dabei beispielsweise ein Substrat, das von der Wirtszelle oder einem oder mehreren anderen anwesenden Enzym(en) in Saccharose umgesetzt wird. Ein zu Saccharose äquivalentes Substrat können auch diejenigen Di- oder Oligosaccharide sein, die von der erfindungsgemäßen Fructosyltransferase alternativ als Substrat genutzt werden können. Hierzu zählt beispielsweise das Trisaccharid Raffinose. Es können aber auch derivatisierte Saccharosen verwendet werden. Das nach einem der vorgenannten Verfahren gewonnene Inulin ist vorzugsweise langkettig und weist vorzugsweise einen Polymerisationsgrad von DP > 20, bevorzugt von DP > 50, insbesondere von DP > 100, und besonders bevorzugt einen Polymerisationsgrad von DP > 200 auf.

Die vorliegende Erfindung betrifft ferner ein Verfahren zur Herstellung von Polyfructose, insbesondere solche des Inulintyps, umfassend den Schritt der Extraktion der Polyfructose aus einer oben beschriebenen erfindungsgemäßen Pflanze(nzelle) und/oder aus Teilen einer solchen Pflanze. Vorzugsweise umfaßt ein solches Verfahren auch den Schritt des Emtens der kultivierten Pflanzen und/oder Teile dieser Pflanzen vor der Extraktion der Polyfructose und besonders bevorzugt ferner den Schritt der Kultivierung erfindungsgemäßer Pflanzen vor dem Ernten. Verfahren zur Extraktion der Polyfructose aus Pflanzen oder Teilen von Pflanzen sind dem Fachmann bekannt und wurden beispielsweise beschrieben von Gibson (International Sugar Journal 96 (1994), 381-387), Vogel (Stud. Plant Sci. 3 (1993), Inulin and Inulin-Containing Crops, 65-75).

Polyfructose, insbesondere solche des Inulintyps, die aus einer erfindungsgemäßen Wirtszelle oder nach einem der vorbeschriebenen erfindungsgemäßen Verfahren erhältlich ist, kann vorzugsweise in der Herstellung von Tensiden, zur Viskositätserhöhung von wäßrigen Systemen, als Detergenz, als suspendierendes Agens, zur Sedimentationsbeschleunigung und Komplexierung oder zur Wasserbindung benutzt werden.

Ferner können die erfindungsgemäßen Wirtszellen, die Polyfructose, insbesondere solche des Inulintyps, synthetisieren, als Nahrungsmittelzusatz verwendet werden. Dies ist vorteilhaft, da Fructane positive Auswirkungen auf die Gesundheit haben (Roberfroid et al., J. of Nutrition 128 (1998), 11-19; Kleesen et al., Am. J. Clin. Nutr. 65 (1997), 1397-1402).

Die vorliegende Offenbarung betrifft ferner ein Verfahren zur Herstellung von Polyfructose, insbesondere solcher des Inulintyps, dadurch gekennzeichnet, daß zur Herstellung der Polyfructose eine pilzliche Fructosyltransferase verwendet wird oder ein Wirtsorganismus, der eine pilzliche Fructosyltransferase exprimiert. Vorzugsweise können dabei erfindungsgemäße Fructosyltransferasen oder erfindungsgemäße Wirtszellen verwendet werden. Die vorliegende Erfindung zeigt erstmals, daß es möglich ist, derartige pilzliche Fructosyltransferasen zur Herstellung von Polyfructose des Inulintyps zu verwenden.

Schließlich betrifft die vorliegende Offenbarung auch die Verwendung pilzlicher Fructosyltransferasen zur Herstellung von Polyfructose, insbesondere solcher des Inulintyps.

Diese und andere Ausführungsformen sind dem Fachmann offenbart und offensichtlich und umfaßt durch die Beschreibung und die Beispiele der vorliegenden Erfindung. Weiterführende Literatur zu einer der oben angeführten Methoden, Mittel und Verwendungen, die im Sinne der vorliegenden Erfindung angewendet werden können, kann dem Stand der Technik entnommen werden, z.B. aus öffentlichen Bibliotheken unter beispielsweise der Benutzung von elektronischen Hilfsmitteln. Zu diesem Zweck bieten sich unter anderem öffentliche Datenbanken an wie die "Medline", die über Internet zur Verfügung stehen, z.B. unter der Adresse http://www.ncbi.nlm.nih.gov/PubMed/medline.html. Weitere Datenbanken und Adressen sind dem Fachmann geläufig und können aus dem Internet entnommen werden, z.B. unter der Adresse http://www.lycos.com. Eine Übersicht über Quellen und Informationen zu Patenten bzw. Patentanmeldungen in der Biotechnologie ist in Berks, TIBTECH 12 (1994), 352-364 gegeben.
- **Figur 1**: stellt Konstruktion pSK-as1 zur Transformation von Bakterien dar.
- **Figur 2**: stellt die Konstruktion des Plasmids p112-as1 zur Transformation von Hefezellen dar.
- **Figur 3**: stellt die Konstruktion des Plasmids pA7-as1 zur Transformation von Pflanzenzellen dar.
- **Figur 4**: stellt die Konstruktion des Plasmids p35-as1 zur Transformation von Pflanzen dar.
- **Figur 5**: stellt die Konstruktion des Plasmids p35-s3-as1 zur Transformation von Pflanzen dar.

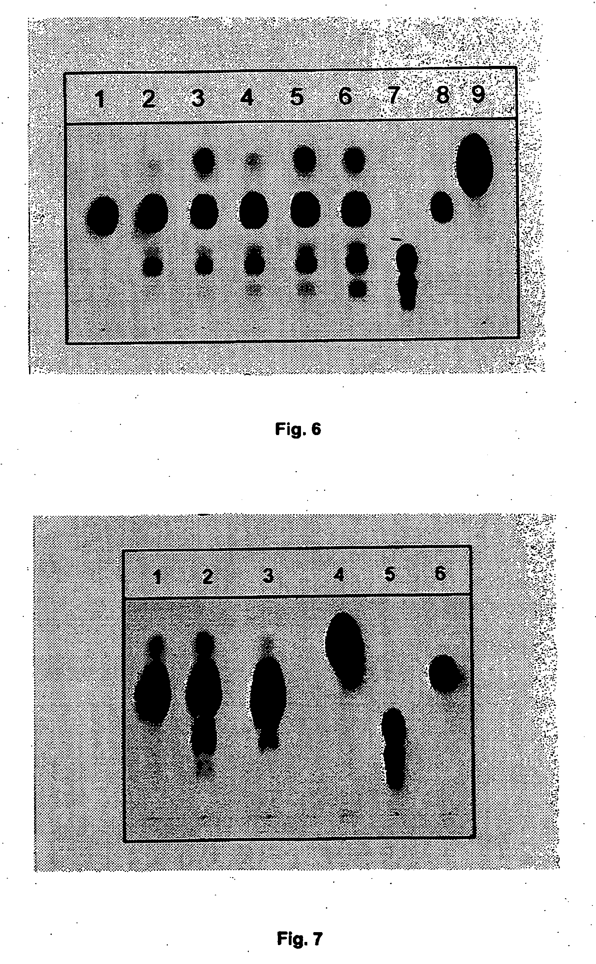

- **Figur 6**: stellt die dünnschichtchromatographische Analyse von mit pSK-as1 transformierten *E*.*coli* dar. Die Spur 1 zeigt einen Kontrollversuch mit dem leeren Vektor pBluescript SK. Die Spur 2 gibt ein Experiment mit dem Plasmid pas1 wieder, bei dem die as1 Codierregion nicht im Leseraster des lacZ Gens ist (Spur 2). In diesem Fall erfolgt die Translation der as1 Codierregion nicht als Fusion an die β-Glucuronidase, sondern erfolgt, mit verminderter Effizienz, vom endogenen Startcodon aus. Die Spuren 3 bis 6 zeigen Versuche mit verschiedenen Transformanden des Konstrukts pSK-as1. Nach Anzucht der Bakterien bis zu einer OD 600 von 0,4 wurden die Kulturen mit 100 mM IPTG induziert. Nach 2h Induktion wurden die Zellen geerntet und in 50 mM Natriumphosphat pH 6,0 lysiert. Proteinextrakte wurden für 12h mit 600 mM Saccharose bei 37°C inkubiert. Als Standard für die Dünnschichtchromatographie wurde in den Spuren 7-9 1-Kestose (7), Saccharose (8) bzw. Fructose (9) aufgetragen.
- **Figur 7**: stellt die dünnschichtchromatographische Analyse von transformierten Hefezellen, enthaltend das Plasmid p112-as1 (Spur 2), bzw. p112-as1L (Spur 3) dar. Der Vektor p112-as1L enthält den 5' Leader des Saccharose-Transporters aus Spinat. Die Spur 1 zeigt einen Kontrollversuch mit nicht transformierten Hefezellen. Fructosyltransferaseaktivität wurde in Proteinextrakten aus den Hefezellen nachgewiesen. Als Standard wurde Fructose (Spur 4), ein Gemisch aus 1-Kestose, Nystose und Fructosyl-Nystose (Spur 5) und Saccharose (Spur 6) aufgetragen.
- **Figur 8**: stellt die dünnschichtchromatographische Analyse von Pflanzenzellen, enthaltend das Plasmid pA7-as1, dar. Spur 1 zeigt eine Transformation mit dem leeren Vektor pA7 (50 µg); die Spuren 2 bis 5 zeigen Transformationen mit dem Vektor pA7-as1 (Spur 2: 10 µg; Spur 3: 20 µg; Spur 4 und 5: 50 µg). Als Standard wurde ein Gemisch aus 1-Kestose, Nystose und Fructosyl-Nystose (Spur 6), Saccharose (Spur 7) und Fructose (Spur 8) aufgetragen.
Für jedes Experiment wurden 500.000 Protoplasten verwendet. Die Protoptasten wurden nach der Transformation zwei Tage bei 25°C inkubiert, anschließend wurde ein Proteinextrakt in 50 mM Natrium-Phosphat pH 6 gewonnen, der für 20 Stunden bei 28°C mit 500 mM Saccharose inkubiert wurde. Aufgetragen wurden 4 µl einer 1/10-Verdünnung des Ansatzes.

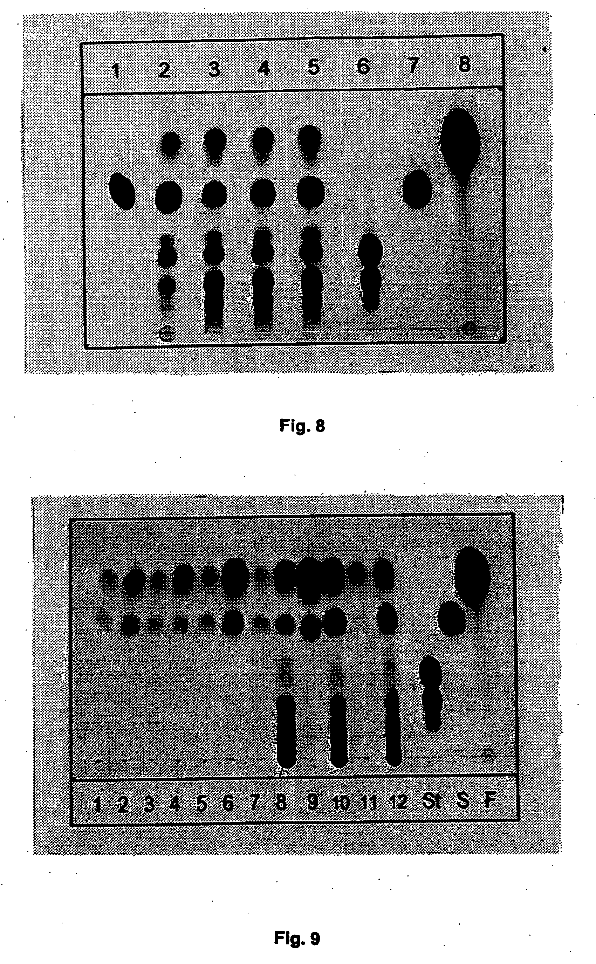

- **Figur 9**: stellt die dünnschichtchromatographische Analyse von Pflanzen dar, die mit dem Konstrukt 35-as1 transformiert wurden. Zwölf Pflanzen wurden zufällig ausgewählt. Jeweils 20 mg Blattmaterial wurde in 200 µl Wasser extrahiert. Vom Extrakt wurden 4 µl aufgetragen. Als Standard wurde Fructose (Spur F), Saccharose (Spur S) und ein Gemisch aus 1-Kestose, Nystose und Fructosyl-Nystose (Spur St) aufgetragen.

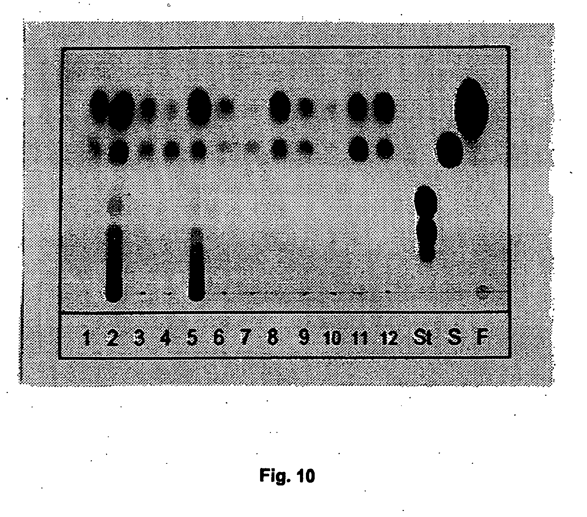

- **Figur 10**: stellt die dünnschichtchromatographische Analyse von Pflanzen dar, die mit dem Konstrukt 35-S3as1 transformiert wurden. Zwölf Pflanzen wurden zufällig ausgewählt. Jeweils 20 mg Blattmaterial wurde in 200 µl Wasser extrahiert. Vom Extrakt wurden 4 µl aufgetragen. Als Standard wurde Fructose (Spur F), Saccharose (Spur S) und ein Gemisch aus 1-Kestose, Nystose und Fructosyl-Nystose (Spur St) aufgetragen.

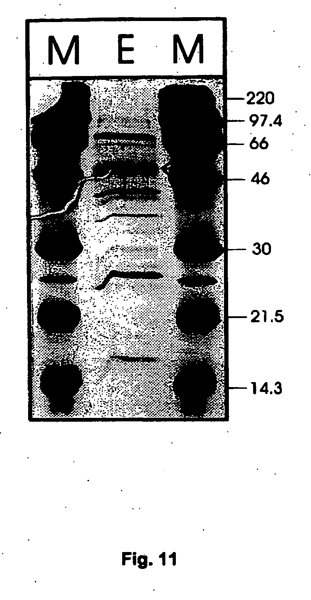

- **Figur 11**: stellt das Eluat "400 bis 0 mM Ammoniumsulfat" einer Phenylsuperose-Säule dar, die mit einem an Fructosyltransferase angereicherten Proteinextrakt aus *Aspergillus sydowi* beschickt worden war (Spur E). In den mit "M" gekennzeichneten Spuren ist ein Größenmarker aufgetrennt. Die Molmassen der Markerproteine sind rechts in kDalton angegeben.

Die Beispiele erläutern die Erfindung.

### Beispiel 1

### Reinigung der af1-SST aus Aspergillus sydowi

*Aspergillus sydowi* IAM 2544 wurde auf einem Kulturmedium angezogen, das 2% Malzextrakt, 0,5% Pepton und 2% Saccharose enthielt. Das Medium wurde durch Zusatz von 2% Agar gefestigt. Sporen wurden ausplattiert, und die Kultur wurde bis zum vollständigen Austrocknen der Platten bei 25°C gehalten. Konidien wurden von den Platten abgeerntet und in 50 mM Natriumphosphat pH 6,0 aufgenommen. Die Lyse der Konidien erfolgte bei drei Passagen durch eine "French Pressure Cell". Zur Reinigung wurde das Homogenat an Sepharose Q adsorbiert. Gebundenes Protein wurde mit einem linearen Gradienten von 0 bis 1000 mM KCl eluiert. Sucrolytisch aktive Fraktionen wurden zwischen 500 und 700 mM KCI erhalten. Diese Fraktionen wurden vereinigt und gegen Natriumphosphat pH 6,0 dialysiert. Zur Anreicherung des Proteins wurde ein weiteres Mal an Sepharose Q (Bettvolumen 2 ml) adsorbiert und in einem Volumen von 10 ml eluiert.
Das Eluat wurde auf 2 M Ammoniumsulfat eingestellt und an Phenylsuperose adsorbiert. Die Elution erfolgte nach Waschen mit 2 M Ammoniumsulfat, 100 mM Natriumphosphat pH 7,0 mit einem linearen Gradienten von 2 M bis 0 M Ammoniumsulfat. Aktive Fraktionen wurden im Elutionsgradienten zwischen 400 und 0 mM Ammoniumsulfat erhalten. Das erhaltene Proteingemisch wurde durch SDS-PAGE analysiert. Das Ergebnis ist in Fig. 11 wiedergegeben. Eine ähnliche Anreicherung einer sucrolytischen Aktivität - allerdings aus Mycel von *Aspergillus sydowi* ist von Muramatsu und Nakakuki (Biosci. Biotech. Biochem. 59 (1995), 208-212) beschrieben. Die Reinigung ergibt kein homogenes Protein, das beispielsweise für eine Sequenzierung geeignet wäre.
Um die Fructosyltransferase zu identifizieren, wurde ein semi-natives Polyacrylamid-Gel eingesetzt, auf das 10 µg Protein des Eluats der Phenylsuperose-Säule in 0,1% SDS, 10% Glyzerin, 50 mM Tris pH 6,8 aufgetragen wurde. Nach der Elektrophorese wurde das Gel in 50 mM Natriumphosphat pH 6,0, 1% Triton X 100 dreimal 10 Minuten umgepuffert und dann 30 Minuten in 50 mM Natriumphosphat pH 6,0, 1 % Triton X 100, 500 mM Saccharose inkubiert. Anschließen wurde das Gel in 0,1% (w/v) 2,3,5,-Triphenyltetrazoliumchlorid (TTC), 0,5 M NaOH gekocht. TTC bildet hierbei mit reduzierenden Zuckern einen roten Formazan-Farbstoff. Die in Fig. 11 markierte Proteinbande ergab eine Färbung aufgrund der sucrolytischen Aktivität des Proteins, das so als Fructosyltransferase von *Aspergillus sydowi* identifiziert werden konnte. Aus einem präparativen Gel wurde die Bande isoliert, das Protein aus dem Gel eluiert und für eine Sequenzierung verwendet. Da das Protein N-terminal blockiert ist, wurden Spaltungen mit Endopeptidase LysC und AspN vorgenommen, die Peptide wurden durch HPLC gereinigt und einer Sequenzierung nach Edmann unterzogen. Hierbei wurden folgende Sequenzen erhalten:

| | | |
|---|---|---|
| LysC: | VLPSTSQASEK | (SEQ ID No. 3) |
| | | |
| AspN: | DDLVTYR | (SEQ ID No. 4) |
| | DPYVFQNHEV | (SEQ ID No. 5) |

Zur Clonierung des Gens wurde eine cDNA-Bibliothek im Phagen Lambda Zap II (Stragene, Heidelberg) angelegt. Da eine Präparation von RNA aus den Konidien nicht möglich war, wurde RNA aus Mycel nach Logemann et al. (Anal. Biochem. 163 (1987), 16-20) präpariert. Poly A⁺-RNA wurde mit dem polyATract System (Promega, Madison, USA) gewonnen. Die Synthese von cDNA und die Clonierung in Lambda Zap II erfolgte nach Herstellerangaben (Stratagene, Heidelberg). Entsprechend der Proteinsequenzen wurden folgende primer entworfen:

| | | |
|---|---|---|
| *primer* asp19down: | 5'-GAYGAYYTNGTNACNTAYMG | (SEQ ID No. 6) |
| *primer* asp19up: | 5'-CKRTANGTNACNARRTCRTC | (SEQ ID No. 7) |
| *primer* asp31-down: | 5'-GTNTTYCARAAYCAYGARG | (SEQ ID No. 8) |
| *primer* asp31up: | 5'-TGRTTYTGRAANACRTANGG | (SEQ ID No. 9) |
| *primer* lys1up: | 5'-GCYTGNSWNGTNSWNGG | (SEQ ID No. 10) |

In einer PCR-Reaktion mit der gesamten cDNA-Bibliothek als Matritze und der primer-Kombination asp19down/asp31up (Annealing-Temperatur 40°C) wurde ein DNA-Fragment von ca. 350 bp erhalten. Dieses Fragment wurde nach radioaktiver Markierung (Megaprime Kit, Boehringer Mannheim, Mannheim) zur Durchmusterung der cDNA-Bibliothek eingesetzt. Erhaltene Clone wurden nach *in vivo*-Excision als pBluescript-Plasmide amplifiziert. Die cDNA-insertionen wurden nach Restriktionsspaltung verglichen, und die Insertion eines Clons wurde vollständig sequenziert. Die Sequenz der cDNA-Insertion ist in SEQ. ID No. 1 wiedergegeben. Die abgeleitete Proteinsequenz ist in SEQ. ID No. 2 wiedergegeben.

### Beispiel 2

Herstellung von Konstrukten, enthaltend Codierregionen pilzlicher Fructosyltransferasen, zur Transformation verschiedener pro- und eukaryontischer Wirtszellen.

Zur Transformation verschiedener Wirtszelle mit pilzlichen Fructosyltransferasen wurde eine Reihe von unterschiedlichen Konstrukten nach molekularbiologischen Standardverfahren (Sambrook et al., 1989, Cold Spring Harbor Laboratory Press) hergestellt. Die Konstrukte sind in Fig. 1 bis 5 dargestellt. Im einzelnen wurden die Konstrukte wie folgt hergestellt:

| | |
|---|---|
| pSK-as1 | ist ein Derivat von pas1, das als *in vivo*-Excision aus einem Lambda Zapll Clon der cDNA-Bank von *Aspergillus sydowi* gewonnen worden war. pas1 enthält die cDNA als EcoRI/XhoI Fragment. pSK-as1 entstand aus pas1 durch Spaltung an BamHI und SmaI, Auffüllen des überstehenden BamHI-Endes und Religation des Vektors. Durch die Entfernung von 4 Nucleotiden wird die Codierregion von as1 in das Leseraster des lacZ-Gens (Figur 1) gebracht. |
| p112-as1 | In den Vektor p112A1 NE (siehe Riesmeier et al., EMBO J. 11 (1992), 4705-4713), der BamHI-geschnitten, aufgefüllt und dann Notl- |
| | geschnitten worden war, wurde das Fragment as1 aus pas1 (Asp718-geschnitten, aufgefüllt und dann Notl-geschnitten) cloniert (Figur 2). |
| pA7-as1 | wurde aus pA7 erzeugt, indem die Codierregion von pas1 als SmaI/Asp718 Fragment, dessen überhängende Enden aufgefüllt worden waren, zwischen die aufgefüllte Asp718 und die SmaI Schnittstelle des Vektors cloniert wurden. Die richtige Orientierung des Fragments wurde durch einen HindIII-Schnitt überprüft, der ein etwa 1900 bp großes Fragment ergab. pA7 ist ein Derivat von pUC18, das zwischen EcoRI und Asp718 den 35S RNA-Promotor des Cauliflower Mosaikvirus (CaMV; 528 bp; nt 6909-7437, Franck et al., Cell 21 (1980), 285-294) enthält, sowie zwischen Sphl und HindIII den Terminator des Octopinsynthase-Gens aus *Agrobacterium tumefaciens* (Gielen et al., EMBO J. 3 (1984), 835-846) (Figur 3). |
| p35-as1 | wurde aus pBinAR erzeugt, indem das Fragment as1 aus pas1 (Asp718/Notl-geschnitten und dann aufgefüllt) in den SmaI-geschnittenen Vektor ligiert wurde. pBinAR ist ein Derivat von pBin19 (Bevan, Nucl. Acids Res. 12 (1984), 8711), das zwischen EcoRI und Asp718 den 35S RNA Promotor des Cauliflower Mosaikvirus (CaMV; 528 bp; nt 6909-7437, Franck et al., a. a. O.) enthält, sowie zwischen Sphl und HindIII den Terminator des Octopinsynthase-Gens aus *Agrobacterium tumefaciens* (Gielen et al., a. a. O.) (Figur 4). |
| p35-s3-as1 | wurde in zwei Schritten kloniert. Zunächst wurde ein BamHI / Asp718 Fragment aus pas1, dessen überhängende Enden mit T4-Polymerase aufgefüllt worden waren, in den Vektor pS3 cloniert, der zuvor mit BamHI geschnitten und aufgefüllt worden war. Hierbei wurde pS3-as1 erhalten. Der Vektor pS3 enthält ein PCR-Fragment des Patatin-Gens B33 (Rosahl et al., Mol. Gen. Genet. 203 (1986), 214-220), das die Nucleotide 725 bis 1400 umfaßt. Das PCR-Fragment ist an nt 725 mit einer Asp718 Schnittstelle versehen (GGTACC), an nt 1400 mit einer Sequenz ATGG, die in Verbindung mit den nt 1399 und 1400 eine NcoI-Schnittstelle ergibt. Das PCR-Fragment ist zwischen die Asp718- und die Smal-Schnittstelle insertiert. Aus pS3-as1 wurde ein Sacl |
| | (aufgefüllt) / XbaI Fragment präpariert, das die Fusion S3-as1 enthält. Dieses Fragment wurde zwischen die Smal- und die XbaI-Schnittstelle von pBinAR cloniert (Figur 5). |

Die Transformation der jeweiligen Wirte erfolgte nach Standardverfahren. *E*. *coli* wurde nach der Methode von Hanahan (J. Mol. Biol. 166 (1983), 557-580) transformiert, *Saccharomyces cerevisiae* wurde nach der Methode von Dohmen et al. (Yeast 7 (1991), 691-692) transformiert, die transiente Genexpression in Tabak-Protoplasten erfolgte nach der Methode von Damm und Willmitzer (Mol. Gen. Genet. 213 (1989), 15-20), die stabile Transformation von Kartoffelpflanzen nach der Methode von Dietze et al. (in: Potrykus, I. and G. Spangenberg (Ed.). Gene transfer to plants. xxii+361 (1995), 24-29; Springer-Verlag: Berlin, Germany; New York, New York, USA. ISBN 3-540-58406-4).

### Beispiel 3

Analyse der Fructosyltransferaseaktivität von transgenen Wirtszellen bzw. -organismen, die pilzliche Fructosyltransferasen exprimieren.

### in vivo Synthese von Inulin

Transgene Wirtszellen oder -organismen, die pilzliche Fructosyltransferasen exprimieren, wurden - soweit es sich nicht um Pflanzengewebe handelt - in Medien mit 2% Saccharose angezogen. Im Falle von *Escherichia coli* K12 als Wirtsorganismus wurde ein funktionales cscB Gen, das die Saccharosepermease von *E*. *coli* codiert, als Konstrukt im Vector pACYC184 eingebracht. Im Falle von *Saccharomyces cerevisiae* wurde das Gen des Saccharosetransporters von Spinat im Vektor p112AINE (Riesmeier et al., EMBO J. 11 (1992), 4705-4713) eingebracht. Die Zellen wurden mindestens 24h in Gegenwart der Saccharose angezogen, anschließend geerntet und nach Waschen in 50 mM Natriumphosphat pH 6,0 aufgeschlossen.
Pflanzen, die die pilzliche Fructosyltransferasen exprimieren, wurden in Erde angezogen. Nach vier Wochen wurden Blatt- oder sonstige Gewebeproben genommen und in 1 ml Wasser/g Frischgewicht in Gegenwart von unlöslichem Polyvinylpolypyrrolidon aufgeschlossen, Zelltrümmer wurden abzentrifugiert.
Jeweils 4 µl der Extrakte wurden auf Kieselgel DC-Fertigfolien (Schleicher und Schüll, Dassel, Germany) aufgetragen und in Aceton/Wasser (87:13) zweimal entwickelt. Der Nachweis von Fructosylresten erfolgte mit einem Harnstoff-Phosphorsäure-Reagenz (Röber et al., Planta 199 (1992), 528-536).

### in vitro Synthese von Inulin

Zellen, die die pilzliche Fructosyltransferasen exprimieren, wurden in 50 mM Natriumphosphat pH 6,0, 50 µM PMSF, 1 mM DTT, 10% (v/v) Ethylenglykol aufgeschlossen. Extrakte aus den Zellen wurden in 50 mM Natriumphosphat pH 6,0, 500 mM Saccharose, 50 µM PMSF, 1 mM DTT, 0,02% (w/v) NaN₃, 10% (v/v) Ethylenglykol für 12h bei 25°C inkubiert. Die Ansätze wurden 1:10 in Wasser verdünnt, dann wurden 4 µl auf Kieselgel DC-Fertigfolien (Schleicher und Schüll, Dassel, Germany) aufgetragen und in Aceton/Wasser (87:13) zweimal entwickelt. Der Nachweis von Fructosylresten erfolgte mit einem Hamstoff-Phosphorsäure-Reagenz (Röber et al., a.a.O.).

Das Ergebnis der jeweiligen Analysen ist in den Fig. 6 bis 10 wiedergegeben. Die Figuren zeigen Kieselgel-Folien nach der dünnschichtchromatographischen Auftrennung von Inkubationsansätzen bzw. Zellhomogenaten und der Anfärbung fructosehaltiger Zucker. Bei der Dünnschichtchromatographie in Aceton/Wasser (87:13) werden die Kohlenhydratmono-, oligo- und polymere nach ihrer Größe aufgetrennt. Fructose wandert hierbei weiter als Saccharose, die wiederum weiter wandert als Kestose usf. Oligomere ab einem DP>7 werden nicht mehr aufgetrennt und verweilen am Auftragsort.

In der Figur 6 sieht man, daß *E*. *coli*-Clone, die mit einem leeren pBluescript-Vektor transformiert sind, Saccharose nicht umwandeln können (Spur 1), während solche, die mit dem Plasmid pas1 transformiert sind, das Trisaccharid Kestose aufbauen. Clone, die mit dem Plasmid pSK-as1 transformiert sind, können auch höhere Oligomere synthetisieren (Spuren 3-6). Gleichzeitig findet eine durch die SFT aus *Aspergillus sydowi* katalysierte Übertragung von Fructoseresten auf Wasser statt, wobei Fructose entsteht. In Figur 7 ist gezeigt, daß Proteinextrakte aus Hefen, die mit dem Plasmid 112-as1 transformiert sind, Fructane aufbauen können. Die Fructosyltransferaseaktivität ist in diesen Hefen größer als in solchen, die mit dem Konstrukt 112-as1L transformiert wurden. Letztere können wegen der geringeren Fructosyltransferaseaktivität innerhalb der im Experiment verfügbaren Zeit nur das Trisaccharid aufbauen. Die Größe der synthetisierten Fructane hängt von der Reaktionsdauer und der Fructosyltransferaseaktivität ab. Die Figur 8 demonstriert, daß die Größe der in Extrakten aus transformierten Tabak-Protoplasten synthetisierten Fructane bei gegebener Reaktionszeit von der Menge der gebildeten Fructosyltransferaseaktivität abhängt, die wiederum von der Menge an transformierten Plasmid pA7-as1 abhängig ist. In den Spuren 3 - 5 sieht man, daß Oligo- und Polymere mit einem DP>7 entstanden sind, die sich in der Chromatographie nicht vom Auftragsort entfernen. Gleiches gilt für Pflanzenextrakte aus stabil transformierten Pflanzen wie in Figur 9 und 10 gezeigt.

### SEQUENZPROTOKOLL

<110> Max-Planck-Gesellschaft zur Förderung der Wissenschaften e.V.
<120> Nucleinsäuremoleküle codierend Enzyme, die Fructosyltransferaseaktivität besitzen und deren Verwendung
<130> C 1056 PCT
<140>
   <141>
<160> 10
<170> PatentIn Ver. 2.1
<210> 1
   <211> 2197
   <212> DNA
   <213> Aspergillus sydowi
<400> 1
   gaattcggca cgaggccgcc atgaagctcc cctcttcact ggacattctt ctcgccagac 60
   aggcggttgg cggtactgag gtcgactacg actcaccacc ccctgacctg acgacgctcc 120
   ctgagaactc gctgttcgag acctggagac ccaagatcca cgttctgccc ccaaatggcc 180
   aaatcgggga cccatgcgct cattacaatg acccggcgac gggtttgttc catgtcggat 240
   tcctccacaa tggcaccggc atttccagcg tctacaccga tgacctggtg acctatcgtg 300
   atatcaatcc taacggcggc tacattattg ttgctggtgg ccccaatgac cccgaagccg 360
   tctttgatgg atctgtcatc cccagcggaa tcgatgacct gcccacgctc ctttatacct 420
   ctgtgacatc gttgccaatc cactggactc taccttatac cccgggaagc gagactcagt 480
   cactggccgt aagtgacgat ggtggtcacc acttcgataa gcttgaccga ggcccagtca 540
   ttccacttcc gccagatgga ctcgatgtta cagccttccg tgacccttat gtattccaga 600
   accacgaggt agacgaagtt accggtagtg acccagatac atggtatgcc gccatatccg 660
   ggggtgtcca tgatgtaggg cccggaatct ttctctaccg caaccaagac tcctcctttg 720
   agaactggga atatctaggc gagtggtggc aagaacccgc caactcgact tggggtgacg 780
   gcacttgggc caaacgctgg ggctacaatt tcgaaaccgg caacgtcttc tctctcgatc 840
   gagaagggta caacgttgac ggccacacgt ttatgactat tggagttgag ggtgcatacg 900
   cgcccatcca gccctcggtt acatctatgc atgccatgct gtgggcagcg ggaaatgttt 960
   cctcagagaa tggcgaaaac gttaccttca cgccttatat ggccggtgct ttggactggg 1020
   gcatggccgc atacgccggt gctggaaagg ttctacccag cacatctcag gcttctgaga 1080
   agagtggagc gcccgaccgc ttcatctcgt gggtttggct tacaggtgat gaatttggtg 1140
   ctgccgctgg atttcctgct gcccagcagg ggtggcagaa tactctcctg cttccgcgtg 1200
   aattgagtat acacacaatc cagaatgtgg tcgacaacga actcatccac gagactgcat 1260
   cctggcgtgt ggcagaacat ggcggcgaga ggagatctgg tggtgtcgag ctggagacac 1320
   tgggaatcaa tattgcgagg gagacctacg atgcaatcgt ctcttctggg acctcgtttg 1380
   aggagccttc gcgagacatt aatgaatccg gcaccattcc atttgagcgc tcgcccacta 1440
   gcaggttctt cgcccttgaa gcccaaatct ccttccccca gtctgcgcga gactcggaag 1500
   tccagtccgg atttcaaatc cttgcttctg aactcgagtg gacgacgatc tattatcagt 1560
   tttcgaatga gtcgattgtc attgaccgta accacacaag tgctgcgtcc gagactacac 1620
   ctggtctcgg tactgtgact gagtctggcc gtatccggct tttcgatatc gcgggtggtt 1680
   gcgatcatga tggacatggc ggccacgatg gcggcaacga tgatgaccac aacggtgacg 1740
   gtgatcatag cggtgacggt gaccacaatg acgatgatga ccataacgtc gacggcgatg 1800
   acaaggagcg tgctcgttac caaaagcgag atggcccttg cgataaagac catgataagg 1860
   ttgagacatt ggatctcacc attgtcgtcg ataactcagt gcttgaagtt tacgccaact 1920
   cacgatttgt ggtgtcgacc tgggttcggc cttggtacac caattcaacg gagattcgct 1980
   tcttccacaa cggcgagggt gaggtcagct ttgacaacat tgcggttcat gatggtctgt 2040
   atgatgcata tccggacagg gacaactgaa gatttcactg gttgatgtat tagcttgcga 2100
   gctataaaga tggcgataat tagtagattt aatccaatga attacctgcc gagattgcag 2160
   atttattctt acaaaaaaaa aaaaaaaaaa actcgag 2197
<210> 2
   <211> 682
   <212> PRT
   <213> Aspergillus sydowi
<400> 2
   Met Lys Leu Pro Ser Ser Leu Asp Ile Leu Leu Ala Arg Gln Ala Val
    1               5                  10                  15
   Gly Gly Thr Glu Val Asp Tyr Asp Ser Pro Pro Pro Asp Leu Thr Thr
               20                  25                  30
   Leu Pro Glu Asn Ser Leu Phe Glu Thr Trp Arg Pro Lys Ile His Val
           35                  40                  45
   Leu Pro Pro Asn Gly Gln Ile Gly Asp Pro Cys Ala His Tyr Asn Asp
       50                  55                  60
   Pro Ala Thr Gly Leu Phe His Val Gly Phe Leu His Asn Gly Thr Gly
   65                  70                  75                  80
   Ile Ser Ser Val Tyr Thr Asp Asp Leu Val Thr Tyr Arg Asp Ile Asn
                   85                  90                  95
   Pro Asn Gly Gly Tyr Ile Ile Val Ala Gly Gly Pro Asn Asp Pro Glu
               100                 105                 110
   Ala Val Phe Asp Gly Ser Val Ile Pro Ser Gly Ile Asp Asp Leu Pro
           115                 120                 125
   Thr Leu Leu Tyr Thr Ser Val Thr Ser Leu Pro Ile His Trp Thr Leu
       130                 135                 140
   Pro Tyr Thr Pro Gly Ser Glu Thr Gln Ser Leu Ala Val Ser Asp Asp
   145                 150                 155                 160
   Gly Gly His His Phe Asp Lys Leu Asp Arg Gly Pro Val Ile Pro Leu
                   165                 170                 175
   Pro Pro Asp Gly Leu Asp Val Thr Ala Phe Arg Asp Pro Tyr Val Phe
               180                 185                 190
   Gln Asn His Glu Val Asp Glu Val Thr Gly Ser Asp Pro Asp Thr Trp
           195                 200                 205
   Tyr Ala Ala Ile Ser Gly Gly Val His Asp Val Gly Pro Gly Ile Phe
       210                 215                 220
   Leu Tyr Arg Asn Gln Asp Ser Ser Phe Glu Asn Trp Glu Tyr Leu Gly
   225                 230                 235                 240
   Glu Trp Trp Gln Glu Pro Ala Asn Ser Thr Trp Gly Asp Gly Thr Trp
                   245                 250                 255
   Ala Lys Arg Trp Gly Tyr Asn Phe Glu Thr Gly Asn Val Phe Ser Leu
               260                 265                 270
   Asp Arg Glu Gly Tyr Asn Val Asp Gly His Thr Phe Met Thr Ile Gly
           275                 280                 285
   Val Glu Gly Ala Tyr Ala Pro Ile Gln Pro Ser Val Thr Ser Met His
   290 295 300
   Ala Met Leu Trp Ala Ala Gly Asn Val Ser Ser Glu Asn Gly Glu Asn
   305 310 315 320
   Val Thr Phe Thr Pro Tyr Met Ala Gly Ala Leu Asp Trp Gly Met Ala
   325 330 335
   Ala Tyr Ala Gly Ala Gly Lys Val Leu Pro Ser Thr Ser Gln Ala Ser
   340 345 350
   Glu Lys Ser Gly Ala Pro Asp Arg Phe Ile Ser Trp Val Trp Leu Thr
   355 360 365
   Gly Asp Glu Phe Gly Ala Ala Ala Gly Phe Pro Ala Ala Gln Gln Gly
   370 375 380
   Trp Gln Asn Thr Leu Leu Leu Pro Arg Glu Leu Ser Ile His Thr Ile
   385 390 395 400
   Gln Asn Val Val Asp Asn Glu Leu Ile His Glu Thr Ala Ser Trp Arg
   405 410 415
   Val Ala Glu His Gly Gly Glu Arg Arg Ser Gly Gly Val Glu Leu Glu
   420 425 430
   Thr Leu Gly Ile Asn Ile Ala Arg Glu Thr Tyr Asp Ala Ile Val Ser
   435 440 445
   Ser Gly Thr Ser Phe Glu Glu Pro Ser Arg Asp Ile Asn Glu Ser Gly
   450 455 460
   Thr Ile Pro Phe Glu Arg Ser Pro Thr Ser Arg Phe Phe Ala Leu Glu
   465 470 475 480
   Ala Gln Ile Ser Phe Pro Gln Ser Ala Arg Asp Ser Glu Val Gln Ser
   485 490 495
   Gly Phe Gln Ile Leu Ala Ser Glu Leu Glu Trp Thr Thr Ile Tyr Tyr
   500 505 510
   Gln Phe Ser Asn Glu Ser Ile Val Ile Asp Arg Asn His Thr Ser Ala
   515 520 525
   Ala Ser Glu Thr Thr Pro Gly Leu Gly Thr Val Thr Glu Ser Gly Arg
   530 535 540
   Ile Arg Leu Phe Asp Ile Ala Gly Gly Cys Asp His Asp Gly His Gly
   545 550 555 560
   Gly His Asp Gly Gly Asn Asp Asp Asp His Asn Gly Asp Gly Asp His
   565 570 575
   Ser Gly Asp Gly Asp His Asn Asp Asp Asp Asp His Asn Val Asp Gly
   580 585 590
   Asp Asp Lys Glu Arg Ala Arg Tyr Gln Lys Arg Asp Gly Pro Cys Asp
   595 600 605
   Lys Asp His Asp Lys Val Glu Thr Leu Asp Leu Thr Ile Val Val Asp
       610                 615                 620
   Asn Ser Val Leu Glu Val Tyr Ala Asn Ser Arg Phe Val Val Ser Thr
   625                 630                 635                 640
   Trp Val Arg Pro Trp Tyr Thr Asn Ser Thr Glu Ile Arg Phe Phe His
                   645                 650                 655
   Asn Gly Glu Gly Glu Val Ser Phe Asp Asn Ile Ala Val His Asp Gly
               660                 665                 670
   Leu Tyr Asp Ala Tyr Pro Asp Arg Asp Asn
           675                 680
<210> 3
   <211> 11
   <212> PRT
   <213> Aspergillus sydowi
<400> 3
   Val Leu Pro Ser Thr Ser Gln Ala Ser Glu Lys
   1               5                   10
<210> 4
   <211> 7
   <212> PRT
   <213> Aspergillus sydowi
<400> 4
   Asp Asp Leu Val Thr Tyr Arg
   1               5
<210> 5
   <211> 10
   <212> PRT
   <213> Aspergillus sydowi
<400> 5
   Asp Pro Tyr Val Phe Gln Asn His Glu Val
   1               5                   10
<210> 6
   <211> 20
   <212> DNA
   <213> Künstliche Sequenz
<220>
   <223> Beschreibung der künstlichen Sequenz: künstliche Sequenz
<400> 6
   gaygayytng tnacntaymg 20
<210> 7
   <211> 20
   <212> DNA
   <213> Künstliche Sequenz
<220>
   <223> Beschreibung der künstlichen Sequenz: künstliche Sequenz
<400> 7
   ckrtangtna cnarrtcrtc 20
<210> 8
   <211> 19
   <212> DNA
   <213> Künstliche Sequenz
<220>
   <223> Beschreibung der künstlichen Sequenz: künstliche Sequenz
<400> 8
   gtnttycara aycaygarg 19
<210> 9
   <211> 20
   <212> DNA
   <213> Künstliche Sequenz
<220>
   <223> Beschreibung der künstlichen Sequenz: künstliche Sequenz
<400> 9
   tgrttytgra anacrtangg 20
<210> 10
   <211> 17
   <212> DNA
   <213> Künstliche Sequenz
<220>
   <223> Beschreibung der künstlichen Sequenz: künstliche Sequenz
<400> 10
   gcytgnswng tnswngg 17

## Patentansprüche

1. Nucleinsäuremolekül, codierend eine Fructosyltransferase, ausgewählt aus der Gruppe bestehend aus
(a) Nucleinsäuremolekülen, die ein Protein codieren, das die unter SEQ ID No. 2 angegebene Aminosäuresequenz umfasst;
(b) Nucleinsäuremolekülen, die die Nucleotidsequenz der unter SEQ ID No. 1 angegebenen codierenden Region umfassen oder eine korrespondierende Ribonucleotidsequenz;
(c) Nucleinsäuremolekülen, die mit dem komplementären Strang eines unter (a) oder (b) genannten Nucleinsäuremoleküls hybridisieren und eine Identität von mindestens 60% zu dem unter (a) oder (b) genannten Nucleinsauremolekül aufweisen; und
(d) Nucleinsäuremolekülen, deren Nucleotidsequenz aufgrund der Degeneration des genetischen Codes von der Sequenz eines unter (c) genannten Nucleinsäuremoleküls abweicht;
sowie die zu diesen komplementären Nucleinsäuremoleküle.

2. Nucleinsäuremolekül nach Anspruch 1, das ein DNA- oder RNA-Molekül ist.

3. Nucleinsäuremolekül nach Anspruch 1 oder 2, das ein Polypeptid aus einem Pilz codiert.

4. Nucleinsäuremolekül nach Anspruch 3, wobei der Pilz aus der Gattung Aspergillus ist.

5. Vektor, enthaltend ein Nucleinsäuremolekül nach einem der Ansprüche 1 bis 4.

6. Vektor nach Anspruch 5, wobei das Nucleinsäuremolekül operativ verknüpft ist mit regulatorischen Elementen, die die Transkription und Synthese einer translatierbaren RNA in pro- und/oder eukaryontischen Zellen gewährleisten.

7. Vektor nach Anspruch 6, wobei das Nucleinsäuremolekül eine Region enthält, die eine Signalsequenz codiert, die die intra- oder extrazelluläre Lokalisation der Fructosyltransferase beeinfluss.

8. Wirtszelle, die ein Nucleinsäuremolekül nach einem der Ansprüche 1 bis 4 oder einen Vektor nach einem der Ansprüche 5 bis 7 enthält, wobei das Nucleinsäuremolekül in Bezug auf die Wirtszelle heterolog ist.

9. Wirtszelle nach Anspruch 8, die eine bakterielle Zelle oder Pilzzelle ist.

10. Pflanzenzelle, die ein Nucleinsäuremolekül nach einem der Ansprüche 1 bis 4 oder einen Vektor nach einem der Ansprüche 5 bis 7 enthält, wobei das Nucleinsäuremolekül in Bezug auf die Pflanzenzelle heterolog ist.

11. Verfahren zur Herstellung einer Pflanze, wobei
(a) eine pflanzliche Zelle genetisch modifiziert wird durch die Einführung eines Nucleinsäuremoleküls nach einem der Ansprüche 1 bis 4 und/oder eines Vektors nach einem der Ansprüche 5 bis 7, wobei das Nucleinsäuremolekül in Bezug auf die Zelle heterolog ist, und
(b) aus einer Zelle eine Pflanze regeneriert wird; und gegebenenfalls
(c) aus der Pflanze nach (b) weitere Pflanzen erzeugt werden.

12. Pflanze enthaltend Pflanzenzellen nach Anspruch 10.

13. Pflanze nach Anspruch 12, die eine Nutzpflanze ist.

14. Vermehrungsmaterial einer Pflanze nach Anspruch 12 oder 13, wobei das Vermehrungsmaterial Pflanzenzelle nach Anspruch 10 enthält.

15. Erntebare Pflanzenteile einer Pflanze nach Anspruch 12 oder 13, wobei die Pflanzenteile Pflanzenzellen nach Anspruch 10 enthalten.

16. Futtermittel und/oder Nahrungsmittel enthaltend erntebare Pflanzenteile nach Anspruch 15.

17. Verfahren zur Herstellung von Wirtszellen nach Anspruch 8 oder 9, wobei geeignete Zellen mit einem Nucleinsäuremolekül nach einem der Ansprüche 1 bis 4 oder mit einem Vektor nach einem der Ansprüche 5 bis 7 transformiert werden.

18. Verfahren zur Herstellung einer Fructosyltransferase, bei dem eine Wirtszelle nach Anspruch 8 oder 9 oder eine Pflanzenzelle nach Anspruch 10, unter Bedingungen kultiviert wird, die die Synthese der Fructosyltransferase erlauben und die Fructosyltransferase aus den kultivierten Zellen und/oder dem Kulturmedium isoliert wird.

19. Fructosyltransferase, codiert durch ein Nucleinsäuremolekül nach einem der Ansprüche 1 bis 4 oder erhältlich nach dem Verfahren nach Anspruch 18.

20. Nucleinsäuremolekül, codierend eine Fructosyltransferase, das spezifisch mit einem Nucleinsäuremolekül nach einem der Ansprüche 1 bis 4 oder einem komplementären Strang davon hybridisiert, wobei die Hybridisierung unter folgenden Bedingungen erfolgt:
Hybridisierung in 50% Formamid, 5 x SSC, 5 x Denhardt's-Lösung, 40 mM Natriumphosphat ph 6,8; 0,5% (Gew./Vol.) BSA, 1% (Gew./Vol.) SDS, 0,1 mg/ml Heringsperma-DNA bei einer Hybridisierungstemperatur von 42°C und anschließendes Waschen der Filter in 0,5 x SSC/0,5% SDS bei 60°C.

21. Verfahrens zur Herstellung von Polyfructose, insbesondere solche des Inulintyps, umfassend:
(a) Kultivierung einer Wirtszelle nach Anspruch 8 oder 9 oder eines eine derartige Zelle enthaltenden Wirtes unter Bedingungen, die die Produktion von Fructosyltransferase und Umsetzung von gegebenenfalls von außen zugeführter Saccharose oder eines äquivalenten Substrats zu Polyfructose erlauben; und
(b) Gewinnung der so hergestellten Polyfructose aus den kultivierten Zellen, dem Wirt oder aus dem Medium.

22. Verfahren zur Herstellung von Polyfructose, insbesondere solche des Inulintyps, umfassend:
(a) Inkontaktbringen von Saccharose oder eines äquivalenten Substrats mit einer Fructosyltransferase nach Anspruch 19 unter Bedingungen, die die Umsetzung zu Polyfructose erlauben; und
(b) Gewinnung der so hergestellten Polyfructose.

23. Verfahren zur Herstellung von Polyfuctose, insbesondere solche des Inulintyps, umfassend den Schritt der Extraktion der Polyfructose aus einer Pflanzenzelle nach Anspruch 10, aus einer Pflanze nacht Anspruch 12 oder 13 oder aus Teilen einer solchen Pflanze.

24. Verfahren zur Herstellung von Tensiden, das die Verfahrensschritte (a) und (b) des Verfahrens nach Anspruch 21 oder 22 oder den Extraktionsschritt des Verfahrens nach Anspruch 23 umfasst.

25. Verwendung von Wirtszellen nach Anspruch 8 oder 9 oder Pflanzenzellen nach Anspruch 10 als Nahrungsmittelzusatz.

26. Verwendung der Fructosyltransferase nach Anspruch 19 zur Herstellung von Polyfructose, insbesondere solche des Inulintyps.

27. Verwendung von Polyfructose, hergestellt durch ein Verfahren nach einem der Ansprüche 21 bis 23, zur Herstellung von Tensiden, zur Viskositätserhöhung von wäßrigen Systemen, als Detergenz, als suspendierendes Agens, zur Sedimentationsbeschleunigung und Komplexierung oder zur Wasserbindung.

## Claims

1. A nucleic acid molecule encoding a fructosyl transferase, selected from the group consisting of
(a) nucleic acid molecules encoding a protein comprising the amino acid sequence depicted under SEQ ID NO:2;
(b) nucleic acid molecules comprising the nucleotide sequence of the coding region depicted under SEQ ID NO:1 or a corresponding ribonucleotide sequence;
(c) nucleic acid molecules hybridising with the complementary strand of a nucleic acid molecule mentioned under (a) or (b) and exhibiting an identity of at least 60% to the nucleic acid molecule mentioned under (a) or (b); and
(d) nucleic acid molecules the nucleotide sequence of which deviates from the sequence of a nucleic acid molecule mentioned under (c) due to the degeneration of the genetic code;
and the nucleic acid molecules complementary thereto.

2. The nucleic acid molecule according to claim 1, which is a DNA or an RNA molecule.

3. The nucleic acid molecule according to claim 1 or 2, encoding a polypeptide from a fungus.

4. The nucleic acid molecule according to claim 3, wherein the fungus is from the genus Aspergillus.

5. A vector containing a nucleic acid molecule according to any one of claims 1 to 4.

6. The vector according to claim 5, wherein the nucleic acid molecule is operatively linked to regulatory elements ensuring the transcription and synthesis of a translatable RNA in pro- and/or eukaryotic cells.

7. The vector according to claim 6, wherein the nucleic acid molecule contains a region encoding a signal sequence influencing the intra- or extracellular localisation of the fructosyl transferase.

8. A host cell containing a nucleic acid molecule according to any one of claims 1 to 4 or a vector according to any one of claims 5 to 7, wherein the nucleic acid molecule is heterologous with regard to the host cell.

9. The host cell according to claim 8, which is a bacterial cell or a fungal cell.

10. A plant cell containing a nucleic acid molecule according to any one of claims 1 to 4 or a vector according to any one of claims 5 to 7, wherein the nucleic acid molecule is heterologous with regard to the plant cell.

11. A method for the production of a plant, wherein
(a) a plant cell is genetically modified by the introduction of a nucleic acid molecule according to any one of claims 1 to 4 and/or a vector according to any one of claims 5 to 7, wherein the nucleic acid molecule is heterologous with regard to the cell, and
(b) a plant is regenerated from a cell; and optionally
(c) further plants are produced from the plant according to (b).

12. A plant containing plant cells according to claim 10.

13. The plant according to claim 12, which is a useful plant.

14. Propagation material of a plant according to claim 12 or 13, wherein the propagation material contains plant cells according to claim 10.

15. Harvestable plant parts of a plant according to claim 12 or 13, wherein the plant parts contain plant cells according to claim 10.

16. Feed stuff and/or food containing harvestable plant parts according to claim 15.

17. A method for the production of host cells according to claim 8 or 9, wherein suitable cells are transformed with a nucleic acid molecule according to any one of claims 1 to 4 or with a vector according to any one of claims 5 to 7.

18. A method for the production of a fructosyl transferase, wherein a host cell according to claim 8 or 9 or a plant cell according to claim 10 is cultivated under conditions allowing for the synthesis of the fructosyl transferase and the fructosyl transferase is isolated from the cultivated cells and/or the culture medium.

19. A fructosyl transferase encoded by a nucleic acid molecule according to any one of claims 1 to 4 or obtainable according to the method of claim 18.

20. A nucleic acid molecule encoding a fructosyl transferase, which specifically hybridises with the nucleic acid molecule according to any one of claims 1 to 4 or a complementary strand thereof, wherein the hybridisation takes place under the following conditions:
hybridisation in 50% formamide, 5 x SSC, 5 x Denhardt's solution, 40 mM sodium phosphate pH 6.8; 0.5% (w/v) BSA, 1% (w/v) SDS, 0.1 mg/ml herring sperm DNA at a hybridisation temperature of 42°C and subsequent washing of the filters in 0.5 x SSC/0.5% SDS at 60°C.

21. A method for the production of polyfructose, in particular polyfructose of the inulin type, comprising:
(a) cultivation of a host cell according to claim 8 or 9 or of a host containing such a cell, under conditions allowing for the production of fructosyl transferase and the conversion from sucrose optionally added externally or of an equivalent substrate to polyfructose; and
b) recovery of the polyfructose produced in that manner from the cultured cells, the host or the medium.

22. The method for the production of polyfructose, in particular polyfructose of the inulin type, comprising:
(a) contacting sucrose or an equivalent substrate with a fructosyl transferase according to claim 19 under conditions allowing for the conversion to polyfructose; and
(b) recovery of the polyfructose produced in that manner.

23. A method for the production of polyfructose, in particular polyfructose of the inulin type, comprising the step of extracting the polyfructose from a plant cell according to claim 10, from a plant according to claim 12 or 13 or from parts of such a plant.

24. A method for the production of tensides comprising the method steps (a) and (b) of the method according to claim 21 or 22 or the extraction step of the method according to claim 23.

25. Use of host cells according to claim 8 or 9 or plant cells according to claim 10 as food additive.

26. Use of the fructosyl transferase according to claim 19 for the production of polyfructose, in particular polyfructose of the inulin type.

27. Use of polyfructose produced by a method according to any one of claims 21 to 23 for the production of tensides, for viscosity increase of aqueous systems, as detergent, as suspension agent, for sedimentation acceleration and complexing or for water binding.

## Revendications

1. Molécule d'acide nucléique, codant une fructosyle transférase, choisie parmi le groupe consistant en :
(a) des molécules d'acide nucléique, qui codent une protéine, qui comprend la séquence des acides aminés donnée par SEQ ID N°2 ;
(b) des molécules d'acide nucléique, qui comprennent la séquences des nucléotides de la région codante donnée par SEQ ID N°1, ou une séquence de ribonucléotide correspondante ;
(c) des molécules d'acide nucléique, qui s'hybrident avec le brin complémentaire d'une molécule d'acide nucléique citée en (a) ou en (b) et qui présentent une identité d'au moins 60% avec la molécule d'acide nucléique citée en (a) ou en (b) ;
(d) des molécules d'acide nucléique, dont la séquence des nucléotides s'écarte de la séquence d'une des molécules d'acide nucléique citée en (c) par la dégénérescence du code génétique ;
ainsi que les molécules d'acide nucléique, complémentaires à celles-ci.

2. Molécule d'acide nucléique selon la revendication 1, qui est une molécule d'ADN ou d'ARN.

3. Molécule d'acide nucléique selon la revendication 1 ou 2, qui code un polypeptide d'un champignon.

4. Molécule d'acide nucléique selon la revendication 3, où le champignon est du genre *Aspergillus*.

5. Vecteur contenant une molécule d'acide nucléique selon l'une quelconque des revendications 1 à 4.

6. Vecteur selon la revendication 5, où la molécule d'acide nucléique est reliée de manière fonctionnelle à des éléments régulateurs, qui garantissent la transcription et la synthèse d'un ARN traduisible dans des cellules pro- et/ou eucaryotes.

7. Vecteur selon la revendication 6, où la molécule d'acide nucléique contient une région qui code une séquence signal, qui influence la localisation intra- ou extracellulaire de la fructosyle transférase.

8. Cellule hôte, qui contient une molécule d'acide nucléique selon l'une quelconque des revendications 1 à 4 ou un vecteur selon l'une quelconque des revendications 5 à 7, où la molécule d'acide nucléique est hétérologue par rapport à la cellule hôte.

9. Cellule hôte selon la revendication 8, qui est une cellule bactérienne ou une cellule de champignon.

10. Cellule végétale, qui contient une molécule d'acide nucléique selon l'une quelconque des revendications 1 à 4 ou un vecteur selon l'une quelconque des revendications 5 à 7, où la molécule d'acide nucléique est hétérologue par rapport à la cellule végétale.

11. Procédé de préparation d'une plante, où
(a) une cellule végétale est modifiée génétiquement par introduction d'une molécule d'acide nucléique selon l'une quelconque des revendications 1 à 4 et/ou un vecteur selon l'une quelconque des revendications 5 à 7, où la molécule d'acide nucléique est hétérologue par rapport à la cellule, et
(b) une plante est régénérée à partir d'une cellule, et le cas échéant,
(c) d'autres plantes sont produites à partir de la plante de (b).

12. Plante contenant des cellules végétales selon la revendication 10.

13. Plante selon la revendication 12, qui est une plante utile.

14. Matériau de multiplication d'une plante selon la revendication 12 ou 13, où le matériau de multiplication contient des cellules végétales selon la revendication 10.

15. Parties de plante à récolter d'une plante selon la revendication 12 ou 13, où les parties de plante contiennent des cellules végétales selon la revendication 10.

16. Fourrage et/ou produit alimentaire contenant les parties de plante à récolter selon la revendication 15.

17. Procédé de préparation de cellules hôte selon la revendication 8 ou 9, où les cellules appropriées sont transformées avec une molécule d'acide nucléique selon l'une quelconque des revendications 1 à 4 ou un vecteur selon l'une quelconque des revendications 5 à 7.

18. Procédé de préparation d'une fructosyle transférase, dans lequel une cellule hôte selon la revendication 8 ou 9 ou une cellule végétale selon la revendication 10 est cultivée dans des conditions qui permettent la synthèse de la fructosyle transférase et la fructosyle transférase est isolée des cellules cultivées et/ou du milieu de culture.

19. Fructosyle transférase, codée par une molécule d'acide nucléique selon l'une quelconque des revendications 1 à 4 ou accessible par le procédé selon la revendication 18.

20. Molécule d'acide nucléique, codant une fructosyle transférase, qui s'hybride spécifiquement avec une molécule d'acide nucléique selon l'une quelconque des revendications 1 à 4 ou un brin complémentaire de celle-ci, où l'hybridation est réalisée dans les conditions suivantes :
hybridation dans le formamide à 50%, 5 x SSC, 5 x solution de Denhardt, phosphate de sodium 40 mM pH 6,8; BSA à 0,5% (poids/volume), SDS à 1% (poids/volume), 1 mg/ml d'ADN de sperme de hareng à une température d'hybridation de 42°C et ensuite, lavage des filtres dans 0,5 x SSC/SDS à 0,5% à 60°C.

21. Procédé de préparation de polyfructose, en particulier celui de type inuline, comprenant :
(a) la culture d'une cellule hôte selon la revendication 8 ou 9 ou d'un hôte contenant une telle cellule, dans des conditions qui permettent la production de la fructosyle transférase et la transformation du saccharose, le cas échéant amené de l'extérieur, ou d'un substrat équivalent en polyfructose, et
(b) obtention du polyfructose ainsi préparé depuis les cellules cultivées, l'hôte ou le milieu.

22. Procédé de préparation de polyfructose, en particulier celui de type inuline, comprenant :
(a) la mise en contact de saccharose ou d'un substrat équivalent avec une fructosyle transférase selon la revendication 19, dans des conditions qui permettent la transformation en polyfructose, et
(b) obtention du polyfructose ainsi préparé.

23. Procédé de préparation de polyfructose, en particulier celui de type inuline, comprenant l'étape d'extraction du polyfructose à partir d'une cellule végétale selon la revendication 10, depuis une plante selon la revendication 12 ou 13 ou de parties d'une telle plante.

24. Procédé de préparation de tensioactifs, qui comprend les étapes (a) et (b) du procédé selon la revendication 21 ou 22 ou l'étape d'extraction du procédé selon la revendication 23.

25. Utilisation de cellules hôte selon la revendication 8 ou 9 ou de cellules végétales selon la revendication 10, comme additif alimentaire.

26. Utilisation de la fructosyle transférase selon la revendication 19, pour la préparation de polyfructose, en particulier celui de type inuline.

27. Utilisation de polyfructose, préparé par un procédé selon l'une quelconque des revendications 21 à 23, pour la préparation de tensioactifs, pour augmenter la viscosité de systèmes aqueux, comme détergent, comme agent de mise en suspension, pour accélérer la sédimentation et la complexation ou pour lier l'eau.
